# EUROPEAN PATENT APPLICATION

(11) **EP 3 967 324 A1**
(43) Date of publication of application: **16.03.2022**
(21) Application number: 21178672.8
(22) Date of filing: 19.07.2017
(51) Int. Cl.: A61K 39/00, C12N 15/10, C12Q 1/68

(54) **SELECTING NEOEPITOPES AS DISEASE-SPECIFIC TARGETS FOR THERAPY WITH ENHANCED EFFICACY**

(30) Priority: 20.07.2016 WO PCT/EP2016/067348
(62) Divisional of application: 17743311.7
(71) Applicant: BioNTech SE, 55131 Mainz (DE)
(72) Inventor: TADMOR, Arbel D., 55294 Bodenheim (DE); SAHIN, Ugur, 55116 Mainz (DE)
(74) Representative: Schnappauf, Georg

(57) **Abstract**

The present invention relates to methods for determining whether neoepitopes that are only expressed in or on diseased cells are suitable disease-specific targets, such that the diseased cell is less likely to be able to escape immune surveillance, and use of the neoepitopes in providing an immune response against diseased cells expressing the neoepitopes.

SEQUENCE LISTING

<110>  BioNTech RNA Pharmaceuticals GmbH

<120>  Selecting Neoepitopes as Disease-Specific Targets for Therapy
       with Enhanced Efficacy

<130>  755-2 PCT2EPT1

<140>
<141>  2017-07-19

<150>  PCT/EP2016/067348
<151>  2016-07-20

<160>  2

<170>  PatentIn version 3.5

<210>  1
<211>  15
<212>  PRT
<213>  Artificial Sequence

<220>
<223>  Linker sequence


<220>
<221>  REPEAT
<222>  (1)..(3)
<223>  Portion of sequence repeated a times, wherein a is independently
       a number selected from 0, 1, 2, 3, 4, 5, 6, 7, 8, 9, 10, 11, 12,
       13, 14, 15, 16, 17, 18, 19, or 20

<220>
<221>  MISC_FEATURE
<222>  (1)..(15)
<223>  a + b + c + d + e are different from 0 and preferably are 2 or
       more, 3 or more, 4 or more or 5 or more

<220>
<221>  REPEAT
<222>  (4)..(6)
<223>  Portion of sequence repeated b times, wherein b is independently
       a number selected from 0, 1, 2, 3, 4, 5, 6, 7, 8, 9, 10, 11, 12,
       13, 14, 15, 16, 17, 18, 19, or 20

<220>
<221>  REPEAT
<222>  (7)..(9)
<223>  Portion of sequence repeated c times, wherein c is independently
       a number selected from 0, 1, 2, 3, 4, 5, 6, 7, 8, 9, 10, 11, 12,
       13, 14, 15, 16, 17, 18, 19, or 20

<220>
<221>  REPEAT
<222>  (10)..(12)
<223>  Portion of sequence repeated d times, wherein d is independently
       a number selected from 0, 1, 2, 3, 4, 5, 6, 7, 8, 9, 10, 11, 12,
       13, 14, 15, 16, 17, 18, 19, or 20

<220>

<221>  REPEAT
<222>  (13)..(15)
<223>  Portion of sequence repeated e times, wherein e is independently
       a number selected from 0, 1, 2, 3, 4, 5, 6, 7, 8, 9, 10, 11, 12,
       13, 14, 15, 16, 17, 18, 19, or 20

<400>  1

Gly Gly Ser Gly Ser Ser Gly Gly Gly Ser Ser Gly Gly Ser Gly
1               5                   10                  15


<210>  2
<211>  9
<212>  PRT
<213>  Artificial Sequence

<220>
<223>  linker sequence

<400>  2

Gly Gly Ser Gly Gly Gly Gly Ser Gly
1               5

## Description

### TECHNICAL FIELD OF THE INVENTION

The present invention relates to methods for determining the suitability of a disease-specific neoepitope as a disease-specific target and the use of such identified suitable neoepitopes in immunotherapy targeted specifically to a patient's diseased tissue, such as tumor tissue, which expresses one or more of the identified suitable neoepitopes.

### BACKGROUND OF THE INVENTION

Cancer is a primary cause of mortality, accounting for 1 in 4 of all deaths. The treatment of cancer has traditionally been based on the law of averages - what works best for the largest number of patients. However, owing to the molecular heterogeneity in cancer, often less than 25% of treated individuals profit from the approved therapies. Individualized medicine based on tailored treatment of patients is regarded as a potential solution to low efficacies and high costs for innovation in drug development.

Personalized cancer immunotherapies are emerging as a potential breakthrough in cancer treatment with the potential to transform the standard of care for the millions of cancer patients yearly diagnosed world-wide. The uniting aspect of personalized cancer immunotherapies is enabling the immune system to target genetic abnormalities (mutations) unique to a patient's cancer. Such disease-specific mutations can encode for neoepitopes, which neoepitopes are disease-specific targets. The most prevalent genetic abnormalities that plague cancer genomes that can be used as disease-specific targets for personalized immunotherapies are nonsynonymous single nucleotide variations (SNVs). Therefore, precise and exhaustive identification of a patient's SNVs in the coding regions of the genome is a critical step in the process of producing personalized cancer immunotherapies.

However, as described herein, knowing the identity of the disease-specific mutation is only part of the picture. Rather, full genetic profiling of a mutation requires knowledge of the exact number of copies of the gene containing the mutation in the diseased cell, *e.g.,* in the tumor cell (including both the wild-type and mutated alleles), the number of copies of the mutated allele in the tumor cell (referred to here as the zygosity of the mutation), and the degree of subclonality of the mutation in a sample of diseased cells, such as a tumor sample. Indeed, copy number variations occurring in diseased cells are an important component of genetic variation in the diseased cells across most disease indications. Moreover, the extent of copy number variations, the identity of genes affected by copy number variations and the precise genetic makeup of copy number variations is unique to each individual and can vary widely from one individual to another. See, generally, Shlien and Malkin, 2009, Genome Med. 1:62; Yang et al., 2013, Cell 153:919-929. Precise knowledge of these genetic features may be critical for selecting mutations that when targeted would be immune to tumor escape, and therefore have the potential to confer total tumor control.

In order to maximize the efficacy of personalized cancer immunotherapies and confer lasting tumor control for the majority of treated patients, therapies need to circumvent in some way the ability of tumors to escape immune surveillance, for example by silencing expression of the mutated target, e.g., by deleting the gene. Without addressing this problem, immunotherapies run the risk of relapse since the immunotherapy cannot target mutations if they are not expressed, e.g., deleted from the genome. Selecting suitable neoepitopes that enhance tumor control would benefit all personalized immunotherapy approaches that target neoepitopes, no matter how they are implemented. Thus, there is a need in the art for ways in which to select neoepitopes resulting from disease-specific mutations that result in enhanced tumor control.

### DESCRIPTION OF INVENTION

### SUMMARY OF THE INVENTION

The present invention provides ways to overcome the deficiencies in the state of the art by providing methods for determining the suitability of a neoepitope resulting from a disease-specific mutation in a gene as a disease-specific target from which a diseased tissue cannot easily escape immune surveillance, which in the case of cancer will result in enhanced tumor control. Once suitable neoepitopes have been identified, such suitable epitopes can be used as disease-specific targets to induce a specific immune response in a patient having the disease. For example, the disease can be cancer and potentially the primary tumor as well as tumor metastases expressing the suitable neoepitope can be targeted for a more effective treatment.

The present invention relates to a method for determining the suitability of a neoepitope resulting from a disease-specific mutation at an allele in a gene (mutated allele) as a disease-specific target comprising determining, in a diseased cell or population of diseased cells, the copy number of the mutated allele encoding the neoepitope. As used herein, copy number can also be referred to as zygosity such that, for example, where the copy number of the mutated allele is 4, the mutated allele has a zygosity of 4. As used herein, an allele is a site in the genome having a specific nucleotide identity, which identity can be the same on both the maternal and paternal copies of the genome (homozygous genotype) or the identity can be different on the maternal and paternal copies of the genome (heterozygous genotype). A mutated allele is an allele, which due to a disease-specific mutation, has a different identity from that site in a corresponding normal genome, *e.g.,* a genome from a non-diseased cell of the same individual (matched genome), preferably from a non-diseased cell of the same tissue type as the diseased cell. A neoepitope suitable as a disease-specific target (suitable neoepitope) as used herein is a neoepitope, which when targeted by the immune system, is less likely to have its expression down-regulated or silenced (*e.g.,* due to deletion) by the diseased tissue such that the diseased tissue is less likely to be able to escape a response, preferably an immunological response generated against the neoepitope by, for example, vaccination against the neoepitope or administering immune cells that are able to target (bind) the neoepitope. In an embodiment, the copy number of the mutated allele can be the same as the copy number of the gene comprising the mutated allele such that the present invention also relates to a method for determining the suitability of a neoepitope resulting from a disease-specific mutation in a gene as a disease-specific target comprising determining, in a diseased cell or population of diseased cells, the copy number of the gene having the disease-specific mutation.

In one embodiment, a high copy number of the mutated allele or gene having the disease-specific mutation indicates the suitability of the neoepitope as a disease-specific target, such that the higher the copy number of the mutated allele or gene having the disease-specific mutation, the higher the suitability of the neoepitope as a disease-specific target. In one embodiment, where the copy number of the mutated allele or gene having the disease-specific mutation in the diseased cell is greater than 2, this indicates the suitability of the neoepitope as a disease-specific target. In one embodiment, where the copy number of the gene having the disease-specific mutation is greater than 3, 4, 5, 6, 7, 8, 9, 10, 20, 30, 40, 50, 60, 70, 80, 90, or is greater than 100, this indicates the suitability of the neoepitope as a disease-specific target.

In cases where not all copies of the gene, in which at least one copy has the mutated allele, have the mutation, it is preferable that many copies of the gene have the mutated allele, *i.e*., it is preferable that a higher rather than lower fraction of the copies of the gene has the mutated allele (higher rather than lower fractional zygosity). Thus, in certain embodiments, the mutated allele is found in a high fraction of copies of the gene of which at least one copy has the mutated allele (fractional zygosity), where the fractional zygosity is the ratio of the copy number of the mutated allele (zygosity of the mutated allele) over the total number of copies of the nucleotide site to which the mutated allele maps, in particular to a reference genome or a corresponding wild-type genome or a matched genome, *i.e.,* wild-type genome from the same individual. The higher the fractional zygosity of copies of the mutated allele, the higher the suitability of the neoepitope as a disease-specific target. Preferably, the fractional zygosity can be greater than 0.2, 0.3, 0.4, 0.5, 0.6, 0.7, 0.8, 0.9, 0.95, and most preferably the fractional zygosity is 1, *i.e.,* that all the copies of the gene in the diseased cell have the mutated allele. In cases where the fractional zygosity is 1, there are no wild-type copies of the gene such that the diseased cell cannot revert back to expressing the corresponding wild-type epitope. As used herein, a fraction of 1 is where the hypothesis that the genetic configuration of the mutated allele/gene, e.g., the copy number, zygosity, is the same cannot be refuted by the data, *i.e.,* is statistically consistent.

It is known that diseased tissue, such as tumors, can be heterogeneous in their genetic make-up and gene expression such that it is possible that not all of the diseased cells in the diseased tissue have the same copy number of a gene and/or of the gene of which at least one copy has the mutated allele (total copy number of the gene) and/or copies of the gene having the mutated allele, much less the disease-specific mutation itself. Therefore, it is preferable that the copy number, e.g., of the mutated allele and/or the fractional zygosity and/or the total number of copies of the nucleotide site to which the mutated allele maps is found to be the same or similar in a high fraction of diseased cells rather than a low fraction of diseased cells in the diseased tissue (a high rather than a low clonal fraction). The higher the fraction of diseased cells having the same or similar copy number, e.g., of the mutated allele and/or the fractional zygosity and/or the total number of copies of the nucleotide site to which the mutated allele maps, the higher the suitability of the neoepitope as a disease-specific target. For example, the clonal fraction can be at least 0.3, 0.4, 0.5, 0.6, 0.7, 0.8, or at least 0.9. In a preferred embodiment, all of the diseased cells in the diseased tissue have the same or similar copy numbers, *i.e*., the clonal fraction is 1, *i.e*., is statistically consistent. As used herein, the same or similar copy number encompasses the same copy number or a copy number within 30%, 25%, 20%, 15%, 10%, 5%, 4%; 3%, 2% or less of the copy number, e.g., copy number or absolute copy number with or without error correction.

Preferably, a clonal fraction of a mutation can be given by the fraction of diseased cells that have the same or similar genetic configuration of the mutation, wherein a genetic configuration of the mutation comprises the total number of copies of the nucleotide site to which the mutation maps and the copy number of the mutated allele. A characteristic is said to be fixed in the population of diseased cells if the characteristic is present in all diseased cells to a degree that cannot be statistically refuted by available data. Preferably, a clonal fraction of 1 means that the genetic configuration of the mutation is fixed in the population of diseased cells. Preferably, the genetic configuration of a mutation is fixed in the population of diseased cells if the mutation is fixed in the population of diseased cells and the CNV affecting the site encoding the mutation is fixed in the population of diseased cells. Preferably, if a mutation for which the total number of copies of the nucleotide site to which the mutation maps is 2 and is in a balanced region of the diseased (tumor) genome, is determined to be fixed in the population of diseased cells, then the genetic configuration of the mutation is fixed.

The gene in which the disease-specific mutation is found can be potentially in any gene in the genome. A preferred type of gene in which a mutation that results in a suitable neoepitope is found is a gene whose expression results in transformation of the cell into a cancerous phenotype or whose lack of expression results in a cancerous cell losing its cancerous phenotype, *i.e.,* a gene whose expression contributes to tumor progression. Such genes are known as driver genes. Examples of driver genes for many type of tumors are well known. For example, a list of 291 high-confidence cancer driver genes acting on 3,205 tumors from 12 different cancer types is disclosed in Tamborero et al., 2013, Comprehensive identification of mutational cancer driver genes across 12 tumor types, Scientific Reports 3:2650. Further driver genes have been identified using the methods disclosed in Youn et al., 2011, Identifying cancer driver genes in tumor genome sequencing studies, Bioinformatics 27(2):175-181, in Sakopamig et al., 2015, Identification of constrained cancer driver genes based on mutation timing, PLoS Comput. Biol. 11(1):e1004027, and in Forbes et al., 2008, Current protocols in human genetics 10-11. The disease-specific mutation in the driver gene may or may not contribute to the cancerous phenotype. Preferably, every copy of the driver gene found in the diseased cell has the disease-specific mutation. Also preferably, all cells in the diseased tissue are diseased cells in which every copy of the driver gene has the disease-specific mutation.

Another preferred type of gene is an essential gene. In an embodiment, an essential gene is a gene, which when silenced or its expression is reduced (e.g., by being deleted), at least results in impaired growth or reduced fitness of the cell, preferably a diseased cell. Such genes are termed herein essential genes. In one embodiment, an essential gene is a gene in which there is an at least 10% reduction in growth or reduced fitness of the diseased cell where the gene is silenced or has its expression reduced compared to a cell in which the gene is not silenced nor reduced expression. In one embodiment, the reduction in growth or reduced fitness is at least 20%, 30%, 40%, 50%, 60%, 75%, 80%, 90%, or at least 95%, most preferably the silencing or reduced expression of the essential gene results in lethality of the diseased cell. Preferably, every copy of the essential gene found in the diseased cell has the disease-specific mutation.

Essential genes are well known in the art, for example, a list of essential genes in humans (*e.g.,* in human cell lines or inferred from other organisms) is disclosed in Liao et al., 2008, Proc. Nat. Acad. Sci. USA 105: 6987-6992 and in Georgi et al., 2013, PLoS Genetics 9 (5):e1003484, as well as corresponding orthologs in other eukaryotic organisms such as mouse (Liao et al., 2007, Trends Genet. 23:378-381), fruit fly (Spradling et al., 1999, Genetics 153:135-177), C. *elegans* (Kamath et al., 2003, Nature 421:231-237), zebrafish (Amsterdam et al., 2004, Proc. Natl. Acad. Sci. USA 101:12792-12797), *Arabidopsis thaliana* (Tzafrir et al., 2004, Plant Physiol. 135:1206-1220), yeast (Kim et al., 2010, Nat. Biotechnol. 28:617-623) and so on. A list of essential genes derived from human cancer cell lines is disclosed in Wang et al., 2015, Science 350:1096-1101, and a list of essential genes can be found at the database of essential genes, DEG5.0 (Zhang et al., 2009, Nucleic Acids Res. 37:D455-D458).

Additionally, a list of essential genes whose deletion/silencing significantly reduces the fitness of a cohort of cell lines can be generated empirically from multiple healthy tissues and/or cancer cell lines, which cell lines can be derived from donors or from the patient. Deletion/silencing of genes can be performed experimentally using various molecular biology techniques such as CRISPR technology, RNA interference, and so on, where the survival or fitness of the cell is determined with and without expression of the putative essential gene. A list of essential genes also can be experimentally determined from cells or from a cell line or a list of essential genes can be obtained by bioinformatic approaches. The cells or cell lines can be diseased cells or cell lines (tumor cells or cell lines) or non-diseased (healthy/normal) cells or cell lines, and can be obtained from donors or the patient having the disease. Preferably, the non-diseased cells or cell lines are from the same tissue type as the diseased cell, and more preferably from the same patient. In embodiments where the disease is cancer, the cells or cell lines can be obtained from the primary tumor or from any metastases, if present. Further, a list of essential genes can be essentially the same as the minimal set of genes expressed in a wide variety of tissues in the body. For example, an essential gene is a gene that is expressed in a wide variety of different tissues and is expressed with a RPKM (minimum reads per kilobase of transcript per million mapped reads) threshold greater than 0, preferably, greater than 0.1, 0.5, 1, 2, 3, 4, 5, 10, 20, 25. Such a list of essential genes can be obtained by analyzing the RNA expression data, *(e.g.,* RNAseq) obtained from a panel of cell samples obtained from at least 5, 6, 7, 8, 9, 10, 15, 20, 25 or more different tissues. Moreover, if a tumor cell line from the patient is available, genes in which all copies contain a mutation encoding a neoepitope can be deleted one at a time and the growth rates of each modified cell line measured. Such measurement/analysis can be performed by high-throughput methods known in the art, which allows for the screening of at least one gene at a time, preferably many genes at a time, in order to assess its effect on the fitness of a diseased or non-diseased cell. Such methods also allow for the detection of synthetic sick or lethal combination of genes, discussed below. Briefly, a library of cell lines, each cell line missing one gene, can be used to test the deletion of one or more candidate genes such that the effect on the cell of the deletion of the genes can be determined.

The present invention further relates to a method for determining the suitability of a neoepitope resulting from a disease-specific mutation in a gene as a disease-specific target comprising determining, in a diseased cell or population of diseased cells, the copy number of the gene, *i.e.,* determining the copy number of a gene in which at least one copy of the gene has a disease-specific mutation. In cases where a gene has a high copy number in a diseased cell such as a tumor cell, *e.g*., due to focal amplification, the chances are very good that the gene may be a driver gene. Thus, a high copy number of a gene indicates the suitability of the neoepitope as a disease-specific target, and the higher the copy number of the gene, the higher the suitability of the neoepitope as a disease-specific target. For example, a high copy number can be a copy number greater than 2, 5, 10, 20, 30, 40, 50, 60, 70, 80, 90, or greater than 100. A high copy number can also be a copy number that is at least 50% greater than the copy number of the gene in a corresponding non-diseased cell. A high copy number can also be where the copy number of the gene in which at least one copy has the disease-specific mutation is at least 2x, 3x, 5x, 10x, 15x, 20x, 25x, 30x, 40x, 50x, 60x, 70x, 80x, 90x, or at least 100x greater than the copy number of the gene in a corresponding non-diseased cell. Due to copy number variations that can also be present in the normal genome, the copy number of the gene in the normal genome is not necessarily two. Moreover, it is known that focal amplifications are more often observed in certain diseases than others, such as in glioblastoma where the epidermal growth factor receptor gene is often focally amplified, thus this embodiment is well suited to use in those diseases.

Further, it is preferable that the copy number of the gene is found to be the same or similar in a high fraction of diseased cells rather than a low fraction of diseased cells, such that the higher the fraction of diseased cells having the same or similar copy number, the higher the suitability of the neoepitope as a disease-specific target. In a preferred embodiment, all of the diseased cells in the diseased tissue have the same or similar copy number of the gene in which at least one copy has the disease-specific mutation, *i.e.,* the clonal fraction is 1. As used herein, the same or similar copy number encompasses the same copy number or a copy number within 30%, 25%, 20%, 15%, 10%, 5%, 4%; 3%, 2% or less of the copy number, *e.g.,* copy number or absolute copy number with or without error correction.

Moreover, the gene having a high copy number, in which at least one copy has a disease-specific mutation resulting in a neoepitope, preferably can be a gene whose expression results in transformation of the cell into a cancerous phenotype or whose lack of expression results in a cancerous cell losing its cancerous phenotype, *i.e.,* a driver gene such as those driver genes known in the art, or can be an essential gene, *e.g.,* a gene which when silenced or its expression is reduced, at least results in impaired growth or reduced fitness of the diseased cell.

The present invention also relates to a method for determining the suitability of a neoepitope resulting from a disease-specific mutation in a gene as a disease-specific target comprising determining, in a diseased cell or population of diseased cells, whether the gene having the disease-specific mutation is an essential gene. In one embodiment, the essential gene is a gene which when silenced or its expression is reduced (*e.g.,* by deletion of the gene), at least results in impaired growth or reduced fitness of the diseased cell. In this embodiment, where the gene is an essential gene and all copies of the essential gene have the disease-specific mutation (fractional zygosity of 1) indicates the suitability of the neoepitope as a preferable disease-specific target. In an embodiment, an essential gene is a gene that is expressed in a wide variety of different tissues and is expressed with a RPKM (minimum reads per kilobase of transcript per million mapped reads) threshold greater than 0, preferably, greater than 0.1, 0.5, 1, 2, 3, 4, 5, 10, 20, 25. Preferably, all copies of the essential gene contain the mutation. Further, it is preferable that a high fraction of diseased cells contain copies of the essential gene in which all copies of the essential gene have the disease-specific mutation (a high rather than low clonal fraction), such that the higher the fraction of diseased cells containing copies of the essential gene in which all copies of the essential gene have the disease-specific mutation, the higher the suitability of the neoepitope as a disease-specific target. In a more preferred embodiment, all of the diseased cells in the diseased tissue have the essential gene in which all copies of the essential gene have the disease-specific mutation, *i.e.,* the clonal fraction is 1.

It is known that certain genes, when individually silenced or where their expression is individually reduced, may have only a small effect, if any, on the fitness or the growth ability of the diseased cell. However, it has been observed that when two of such genes both of which have been silenced or where each of their expression has been reduced can result in a much stronger growth impairment, up to lethality. Such genetic combinations are referred to as synthetic lethal or synthetic sick/impaired. See Nijman, 2011, Synthetic lethality: General principles, utility and detection using genetic screens in human cells, FEBS Lett. 585:1-6 for a discussion on synthetic lethal and synthetic sick genes and methods for identifying such genes. Since both genes are required for the cell to survive, it is unlikely that the cell will silence or reduce expression of both of the genes. Thus, a suitable combination of neoepitopes as disease-specific targets can result from disease-specific mutations in at least two genes, which genes together are synthetically lethal or synthetically sick. In view thereof, the present invention further relates to a method for determining the suitability of a combination of at least two neoepitopes resulting from disease-specific mutations in at least two genes as a combination of disease-specific targets comprising determining whether a combination of the at least two genes each having a disease-specific mutation are synthetic lethal or synthetic sick genes. When the combination of the at least two genes results in a synthetic lethal or synthetic sick phenotype, this indicates that the resulting neoepitopes are a suitable combination of disease-specific targets. In a preferred embodiment, synthetic sick results in at least a greater effect on cell growth/fitness that what would be expected from the additive effect of deletion/reduced expression of each gene individually. This approach is favored where there is a high number of suitable neoepitopes since the higher the number of neoepitopes, the greater the number of combinations that could be synthetic sick or lethal. For example, 10 mutations corresponds to 45 possible combinations, 100 mutations corresponds to 4950 combinations, and 1000 mutations corresponds to about 500,000 combinations. In certain embodiments, the at least two genes each have a higher rather than lower fractional zygosity, preferably a fractional zygosity of 1, and/or each have a higher rather than lower clonal fraction, preferably a clonal fraction of 1, both in the diseased cells and diseased cells in the diseased tissue. As used herein, each neoepitope found in a combination of suitable neoepitopes is each considered to be a suitable neoepitope for the purposes of the present invention.

As referred to herein, the copy number of the gene, either in the diseased or non-diseased cell, can be the relative copy number, but is preferably the absolute copy number, and more preferably is the absolute copy number normalized against a ploidy, *e.g.,* the ploidy of the genome of the diseased cell, *i.e.,* the copy number of the genome. Even more preferably, the relative, absolute and normalized copy number is error corrected.

When estimating absolute copy number, for example of a mutated allele or gene or its zygosity the estimation may be inaccurate and correcting the absolute copy number to take into account sources of error is desirous. In embodiments where next generation sequencing is used to obtain sequence information from genomes and exomes, sources of error can include: a bias in the estimated purity of the sample of diseased tissue such as a tumor sample, a bias in any estimated parameter required in order to derive the purity and/or absolute copy numbers, stochastic errors due to the finite coverage of the sample being sequenced, limited detection capability due to a low purity, a low clonal fraction, and so on.

In an embodiment where absolute copy number is determined using balanced heterozygous segments containing a heterozygous SNP, such as that disclosed in International PCT Patent Application entitled "Tumor Modeling Based on Primary Balanced Heterozygous Segments" filed on even date herewith, the disclosure of which is incorporated by reference herein in its entirety, an error in the absolute copy number of a segment can propagate to other estimated parameters, such as the absolute copy number of the mutated allele, *e.g.,* a SNV, encoding a neoepitope, the zygosity of the mutated allele, the clonal fraction and so on. Since such downstream estimated parameters have clinical implications for determining the suitability of a neoepitope as a disease specific target for a patient as described herein, is it desirable to correct errors in the absolute copy number to obtain to most accurate value of the absolute copy number. Moreover, since the mutations encoding the neoepitopes can be prioritized for their suitability to be included in a vaccine to be given to the patient using the criteria discussed herein, and because a fraction zygosity of 1 is qualitatively better than a fraction zygosity smaller than 1, in particular when the gene containing the mutation is an essential gene, it is beneficial to have the most accurate estimates of absolute copy numbers and any parameter derived thereof from.

In a preferred embodiment, the absolute copy number of a mutated allele, *e.g.,* a SNV, and/or the zygosity of a SNV can be error corrected. In an embodiment, the absolute copy number of a SNV is first error corrected, and then the zygosity of the SNV is corrected to reflect the error corrected absolute copy number of the SNV. Once the absolute copy number of the SNV and/or zygosity of the SNV are error corrected, the estimation of the clonal fraction can be also corrected to reflect the error corrected absolute copy numbers, including the determination if the clonal fraction is statistically consistent with a value of 1.

The absolute copy number of a SNV is preferably given by the absolute copy number of the segment to which the SNV maps. The absolute copy numbers of all segments in the diseased, *e.g.,* tumor genome can be error corrected, including the absolute copy number of the SNV. Once the absolute copy numbers of all segments in a genome are error corrected, an error corrected ploidy can be calculated based on the error corrected absolute copy numbers of segments in the diseased, *e.g.,* tumor genome.

In a specific embodiment, if the absolute copy number of a SNV is error corrected such that the new absolute copy number differs from the original absolute copy number this can be taken as an indication that the estimated absolute copy number of the SNV is not reliable.

One example of error correction of absolute copy numbers of segments is parity error correction, comprising correcting an odd absolute copy number of a segment to an even absolute copy number if the segment is in a balanced region. A balanced region is a region of the diseased, *e.g.,* tumor genome wherein the maternal and paternal alleles within the region underwent equal (balanced) amplification, or both maternal and paternal alleles did not undergo any amplification at all.

The decision to error correct the odd absolute copy number of the segment to the closest *higher* even absolute copy number or the closest *lower* even absolute copy number can depend on the disease reads and normal reads mapping to the segment, and comparison to the predicted boundaries defining the absolute copy number of a segment. Wherein a normal read is a read pertaining to the sequenced normal sample, and a disease read is a read pertaining to the sequenced diseased sample. In particular, when the disease is cancer, a tumor read is a read pertaining to the sequenced tumor sample.

For example, in a parity error correction of the first kind, if *CNₘᵤₜ* is the absolute copy number in the diseased genome of the segment to which the mutation maps, the absolute copy number of a segment predicted to have a value of *CNₘᵤₜ,* can be corrected to *CNₘᵤₜ* + 1 if *r > ρ^{th},* and to *CNₘᵤₜ* - 1 if r < *ρ^{th},* where r is the disease over normal segment read count ratio (the ratio of the number of disease, e.g., tumor reads mapping to the segment over the number of normal reads mapping to the segment), wherein *ρ^{th}* is the predicted decision boundary, the value of which depends also on the purity of the disease tissue sample, *e.g.,* tumor sample.

An allele specific copy number of a segment is the number of copies in the diseased genome of either the maternal allele or paternal allele of the segment. When the segment contains a heterozygous SNP (heterozygous segment), the heterozygous SNP can be used to determine the allele specific copy number of the segment. A heterozygous segment can be assigned to a preferred node, wherein a node can be defined to be a unique combination of an absolute copy number of the heterozygous segment and an allele specific copy number of heterozygous segment. Even nodes are a subset of nodes for which the absolute copy number of the segment is even. If a heterozygous segment contains more than one heterozygous SNP, the group of two or more heterozygous SNPs can be represented by either a single member of the group, or the allele frequencies of all members of the group can be averaged, or a median can be taken, so long as the allele frequency of each heterozygous SNP is calculated consistently for either the allele having the higher or lower number of copies in the diseased genome.

A parity error correction of the first kind of a heterozygous segment can involve finding the most likely even node to correspond to the heterozygous segment, for example, based on a maximum likelihood framework given the measured disease (e.g., tumor) reads and normal reads mapping to the segment.

In a parity error correction of the second kind, the nearest upstream and downstream segments that do not require parity error correction are identified, preferably within 10Mb, 5Mb, 1Mb of the segment containing the SNV. If the absolute copy number of both nearest upstream and downstream segments are identical, then the absolute copy number of the segment containing the SNV is changed to the absolute copy number of the nearest segment. Generally, parity error correction of the second kind is preferred to parity error correction of the first kind, unless it cannot be implemented because suitable neighboring segments cannot be identified, in which case parity error correction of the first kind can be applied.

Other forms of error correction of an absolute copy number of a segment can also be introduced instead of or in addition to parity error correction. For example, methods considering the absolute copy number of segments in the immediate vicinity of the gene containing the mutation in the diseased genome, wherein the absolute copy number of the segment containing the SNV is changed to the mode of the absolute copy numbers of neighboring segments, preferably if the change in absolute copy number is not more than 3, 2 and preferably 1, and preferably if most of the neighboring segments (50%, 60%, 70%, 80%, 90%, 100%) have an absolute copy number equal to the mode.

Different error correction schemes for the absolute copy numbers can be combined. In a preferred embodiment, first parity error correction is applied as first layer of error correction, on top of which additional error correction methods can be applied.

As used herein, a segment can be a predetermined region of the genome, e.g., predetermined based on a reference genome. A segment can span a gene, *e.g.,* as defined in a reference genome that the reads are aligned to. A segment can also be a fragment of a gene, an exon, a union of exons, or the union of exons associated within a given gene. A segment can also be another set of predetermined regions in a reference genome (with or without introns), or another set of predetermined regions in a reference genome based on the normal genome. In specific embodiments, a segment can be a region of the reference genome with a given constant copy number and/or a given allele specific copy number in the diseased, *e.g.,* tumor, genome or alternatively a fragment of a gene with a given constant copy number and/or allele specific copy number in the diseased, *e.g.,* tumor genome. A segment can be defined to include or to exclude introns.

A number of copies of a segment in a given genome (*e.g.,* in the normal genome, or in the tumor genome) can be defined as how often in total the nucleotide sequence of the segment occurs in the genome, ignoring variations caused by SNPs and/or SNVs and/or other cancer-associated changes such as, but not limited to, mutation, insertions, deletions and/or other cancer-related genetic variants. Preferably the different copies of the segment in the given genome have the same length or nearly the same length.

The number of copies of a segment in a genome can mean the number of physical copies of the segment in a cell containing the genome. An absolute copy number of a segment in the normal genome can be defined as the number of physical copies of the given segment in a healthy cell. An absolute copy number of a segment in the diseased, *e.g.,* tumor, genome can be defined as the number of physical copies of the given segment in a diseased, *e.g.,* tumor, cell. The number of copies of a segment in the genome can be referred to as the absolute copy number of the segment in the said genome. A copy number can mean an absolute copy number.

In a specific embodiment, if only a part of a segment is amplified or deleted in a genome, then such a partial copy of the segment can either be counted as a copy of the segment or not counted as a copy of the segment. In a preferred embodiment, copies of the segment spanning less than 90%, 80%, 70%, 60%, 50%, 40%, 30%, 20%, 10% or 5% of the segment length can be ignored.

A reference genome is used for mapping reads and providing a coordinate system for the normal genome and the diseased, e.g., tumor, genome, wherein a coordinate system can comprise providing a chromosome number, a nucleotide position in the chromosome, as well as directionality of the read, where the position in the chromosome indicated by the line.

A reference genome can be based on the genome of one or more members from the same species as a subject providing the sample of diseased tissue, or can be based on the normal genome of the subject.

A sample of diseased tissue, such as a tumor sample, also can comprise contamination from the normal genome, in particular the normal genome of the same patient from which the sample was taken, and/or in case of intratumor heterogeneity, also comprise more than one tumor genome. The purity, tumor sample purity, tumor purity, and sample purity are all taken to be equivalent terms, preferably meaning the fraction of tumor cells present in a tumor sample. Normal contamination preferably means the fraction of normal cells present in the tumor sample, and can be given by one minus the purity.

Normalizing against the ploidy of the cell controls for the presence of copies of a gene due to genome duplication events. In an embodiment, the absolute copy number can be normalized against the ploidy of the genome, which ploidy is the average of the absolute copy number of all segments in a given genome is a given cell weighted by the length of each segment. In an embodiment, the absolute copy number can be normalized against the ploidy of the chromosome which contains the mutated gene of interest (comprising the mutation), which ploidy is the average of the absolute copy number of all segments on the given chromosome in a given cell weighted by the length of each segment on the chromosome. In an embodiment, the absolute copy number can be normalized against the ploidy of a neighboring region of the chromosome which contains the mutated gene of interest, which ploidy is the average of each segment in the given region in a given cell weighted by the length of each segment in the region. The neighboring region can be within a predetermined distance of the gene having the disease-specific mutation, *e.g.,* within 100 megabases (Mb), 75 Mb, 50 Mb, 25 Mb, 10 Mb, 5 Mb, 4 Mb, 3 Mb, 2 Mb, or 1 Mb of the gene having the disease-specific mutation. The copy number of a segment can be calculated routinely by methods known in the art, both experimentally and computationally. For example, EP Patent Nos. 2 198 292 B1 and EP 2 002 016 B1 disclose methods for determining relative copy number and copy number frequency of nucleic acid sequences, respectively. Further, EP Patent Application Publication No. 2 835 752 A and International Patent Application Publication Nos. WO 2014/014497 and WO 2014/138153 also disclose methods for determining copy number variations. See also, Machado et al., 2013, Copy Number Variation of Fc Gamma Receptor Genes in HIV-Infected and HIV-Tuberculosis Co-Infected Individuals in Sub-Saharan Africa, PLoS, 8(11):e78165. Other methods include the use of FACS, FISH or other fluorescent-based methods, spectral karyotyping (SKY), and digital PCR. A segment can also be a gene.

The disease-specific mutation can be any mutation that results in the expression of a neoepitope, preferably on the surface of the diseased cell. In particular, the mutation can be an indel or gene-fusion event or can be a single nucleotide variation (point mutation). Preferably, the disease-specific mutations are a non-synonymous mutations, preferably non-synonymous mutations of proteins expressed in a tumor or cancer cell. Any method known in the art for determining disease-specific mutations can be used, and in particular methods using next generation sequencing data to determine any changes between the genome/exome of diseased cells compared to the genome/exome of corresponding non-diseased, wild-type cells is preferred. For example, Carter et al., 2012, Absolute quantification of somatic DNA alterations in human cancer, Nature Biotechnology 30:413-421; Cibulskis et al., 2013, Sensitive detection of somatic point mutations in impure and heterogeneous cancer samples, Nature Biotechnology 31:213-219; and Li and Li, 2014, A general framework for analyzing tumor subclonality using SNP array and DNA sequencing data, Genome Biology 15:473-495 disclose methods not only for identifying disease-specific mutations, but also disclose methods for determining gene copy number, fractional zygosity and fractional subclonality of the zygosity and fractional zygosity. Another method for determining copy number, such as absolute copy number, concerns the use of segments of the genome, each segment containing at least one heterozygous single nucleotide polymorphism (SNP), and which segments are balanced (equal number of each version of the heterozygous SNP) and share a common number of copies (primary copy number) which preferably is the most frequently observed absolute copy number of all the balanced segments of the genome, as disclosed in International PCT Patent Application entitled "Tumor Modeling Based on Primary Balanced Heterozygous Segments" filed on even date herewith, the disclosure of which is incorporated by reference herein in its entirety. Moreover, in additional to determining absolute copy number, this application can also determine zygosity, fractional zygosity, and subclonality of the mutated allele or the gene of which at least one copy comprises the mutated allele. Further, the methodology therein also performs error correction for absolute copy numbers, which improves the accuracy of absolute copy numbers and zygosities and parameters derived therein, such as subclonality, ploidy, and so on.

Generally, a total number of copies of the nucleotide site to which the mutated allele maps can mean the absolute copy number of the mutation, which can mean the absolute copy number of the SNV, in particular when the mutation is a SNV. Generally, the absolute copy number of the mutation can preferably be given by the absolute copy number of a segment to which the mutation maps (wherein the absolute copy number is in the diseased, *e.g.,* tumor genome).

Generally, a copy number of a mutated allele encoding a neoepitope can mean an absolute copy number of a mutated allele of a mutation, which can mean an absolute copy number of the alternate allele of a SNV (a zygosity of a SNV), wherein the alternate allele of the SNV is the mutated allele, in particular when the mutation is a SNV.

Preferably, when the mutation is not a SNV, the absolute copy number of a mutated allele of a mutation can be estimated in a similar manner to the method applied for SNVs.

Generally, a copy number of a gene can mean the absolute copy number of a segment, wherein the segment can be the gene, or can encompass the gene. A copy number of a gene can mean an absolute copy number of a gene.

Preferably, the disease can be any disease in which an immune response against the diseased cell/tissue is desired, such as a virally-infected cell. Preferably, the disease is cancer.

The methods of the invention may comprise the further step of determining the usability/appropriateness of the suitable neoepitopes identified by the methods of the invention as suitable disease-specific targets for use in a method to provide an immune response against the suitable neoepitope, such as inclusion of the suitable neoepitope in a cancer vaccine. Thus, further steps can involve one or more of the following: determining the antigenicity and/or immunogenicity of the suitable neoepitope; assessing whether the suitable neoepitope is expressed on the surface of the diseased cell; ability of a peptide comprising the suitable neoepitope to be presented as a MHC presented epitope; determining the efficacy of expression of the suitable neoepitope from an encoding nucleic acid; determining whether the envisaged suitable neoepitopes, in particular when present in their natural sequence context, *e.g*. when flanked by amino acid sequences also flanking said neoepitopes in the naturally occurring protein, and when expressed in antigen presenting cells are able to stimulate T cells such as T cells of the patient having the desired specificity.

Once it has been determined that the neoepitope is suitable/appropriate for use as a target in view of its antigenicity/immunogenicity, ability to be expressed, ability to be presented as a MHC presented epitope, *etc.,* the identified suitable neoepitopes can be ranked, *i.e.,* prioritized, on their potential to not be down-regulated or deleted from the diseased cell, that is less likely that the diseased tissue can escape the targeting of the neoepitope. For example, one prioritization starts with the "best" neoepitope, which is one that is encoded by an essential gene, in which all copies of the essential gene have the mutation encoding the neoepitope, followed by a pair of synthetic sick or lethal genes, in which each copy of each gene has the mutation encoding the neoepitope, followed by a neoepitope encoded by a known driver gene with a very high absolute copy number in which all copies of the gene have the mutation, followed by a neoepitope encoded by a gene that is not known to be a driver gene with a very high absolute copy number and a high zygosity, followed by a neoepitope encoded by a gene with a high copy number (zygosity), and so on.

In an embodiment, a neoepitope encoded by an essential gene with a fractional zygosity of 1 is preferred to other neoepitopes that are not encoded by an essential gene. In an embodiment, between two essential genes encoding neoepitopes with a fractional zygosity of 1, the neoepitope encoded by the gene having a higher absolute copy number is preferred. In an embodiment, between two essential genes encoding neoepitopes, the neoepitope encoded by the gene leading to a lower fitness when deleted is preferred. In an embodiment, between genes encoding neoepitopes in which all the genes have a fractional zygosity of 1, the neoepitopes encoded by the genes having a higher absolute copy number are preferred. In an embodiment where the fractional zygosity is less than 1, then neoepitopes encoded by genes having a high zygosity are preferred to genes having a high fractional zygosity, and if the zygosity is the same or similar, then genes having a high absolute copy number are preferred to those with a high fractional zygosity (10 copies of the mutated allele/20 total copies of the nucleotide site is better than 3/4 due to higher zygosity; 10/100 is better than 10/20 because the former may be a driver gene; 9/100 is better than 10/20 because the zygosity is similar but the former may be a driver gene). In an embodiment, a neoepitope encoded by a driver gene, in which the disease-specific mutation is responsible for transforming the cell into a cancerous phenotype is preferred to those in which the mutation does not have a role in transforming the cell into the cancerous phenotype. Moreover, it is preferred that the neoepitopes have a higher rather than lower clonal fraction.

Further embodiments of the present invention relate to the use of the methods of determining the suitability of a neoepitope as a disease-specific target for the manufacture of a medicament, such as a vaccine, *e.g.,* a personalized cancer vaccine. The vaccine can be derived from one or more suitable neoepitopes or from a combination of suitable neoepitopes identified by the methods of the invention. In a preferred embodiment, the vaccine comprises a peptide or polypeptide comprising one or more suitable neoepitopes or a combination of suitable neoepitopes identified by the methods of the invention, or a nucleic acid encoding said peptide or polypeptide.

In particular, a recombinant vaccine can be provided which when administered to a patient preferably provides a collection of MHC presented epitopes at least one of which is a suitable neoepitope or at least two of which are a suitable combination of neoepitopes identified by the methods of the present invention, such as 2 or more, 5 or more, 10 or more, 15 or more, 20 or more, 25 or more, 30 or more and preferably up to 60, up to 55, up to 50, up to 45, up to 40, up to 35 or up to 30 MHC presented epitopes. Presentation of these epitopes by cells of a patient, in particular antigen presenting cells, preferably results in T cells targeting the epitopes when bound to MHC and thus, the patient's tumor, preferably the primary tumor as well as tumor metastases, expressing antigens from which the MHC presented epitopes are derived and presenting the same epitopes on the surface of the tumor cells.

The methods of the present invention are also useful in the manufacture of recombinant immune cells expressing an antigen receptor targeted to a suitable neoepitope or to one neoepitope in a combination of suitable neoepitopes. Preferably, the immune cells are T cells and the antigen receptor is a T cell receptor.

The present invention also relates to a method for providing a recombinant immune cell targeted to a suitable neoepitope or to one epitope in a combination of suitable neoepitopes, said method comprising transfecting an immune cell with a recombinant antigen receptor targeted to the suitable neoepitope or to the one epitope in a combination of suitable epitopes identified by the methods of the present invention for determining the suitability of a neoepitope as a disease-specific target, as well as to recombinant immune cells produced by such methods.

The present invention also provides methods for targeting a cell population or tissue expressing one or more neoepitopes. For example, an antibody directed against one or more of the neoepitopes can be used to target the cells or tissue expressing the one or more neoepitopes identified by the methods described herein. In one embodiment, the present invention provides methods for providing an immune response to a target cell population or target tissue expressing one or more neoepitopes in a mammal, said method comprising administering to the mammal (a) one or more immune cells expressing one or more antigen receptors targeted to the one or more neoepitopes; (b) administering a nucleic acid encoding one or more of the neoepitopes; or (c) administering a peptide or polypeptide comprising one or more of the neoepitopes, in which the neoepitopes are identified according to the methods of the invention for determining the suitability of a neoepitope as a disease-specific target. In one embodiment, the method for providing an immune response to a target cell population or target tissue expressing one or more neoepitopes in a mammal comprises the steps of (i) determining, in a diseased cell or population of diseased cells, the copy number of a mutated allele in a gene which encodes a neoepitope (a disease-specific mutation); and (ii) administering (a) an immune cell expressing an antigen receptor targeted to the neoepitope resulting from the disease-specific mutation; (b) administering a nucleic acid encoding the neoepitope resulting from the disease-specific mutation; or (c) administering a peptide or polypeptide comprising the neoepitope resulting from the disease-specific mutation.

In one embodiment, the method for providing an immune response to a target cell population or target tissue expressing one or more neoepitopes in a mammal comprises the steps of (i) determining, in a diseased cell or population of diseased cells, the copy number of a gene in which at least one copy of the gene has a disease-specific mutation which results in a neoepitope; and (ii) administering (a) an immune cell expressing an antigen receptor targeted to the neoepitope resulting from the disease-specific mutation; (b) administering a nucleic acid encoding the neoepitope resulting from the disease-specific mutation; or (c) administering a peptide or polypeptide comprising the neoepitope resulting from the disease-specific mutation. In one embodiment, the method for providing an immune response to a target cell population or target tissue expressing one or more neoepitopes in a mammal comprises the steps of (i) determining, in a diseased cell or population of diseased cells, whether a gene having a disease-specific mutation resulting in a neoepitope is an essential gene; and (ii) administering (a) an immune cell expressing an antigen receptor targeted to the neoepitope resulting from the disease-specific mutation; (b) administering a nucleic acid encoding the neoepitope resulting from the disease-specific mutation; or (c) administering a peptide or polypeptide comprising the neoepitope resulting from the disease-specific mutation. Preferably, all copies of the essential gene have the disease-specific mutation, *i.e.,* the fractional zygosity is 1.

In one embodiment, the method for providing an immune response to a target cell population or target tissue expressing one or more neoepitopes in a mammal comprises the steps of (i) determining, in a diseased cell or population of diseased cells, whether a combination of at least two genes, each having a disease-specific mutation resulting in a neoepitope, are synthetic lethal or synthetic sick genes; and (ii) administering (a) one or more immune cells expressing one or more antigen receptors targeted to the one or more neoepitopes resulting from the disease-specific mutations of the at least two genes; (b) administering a nucleic acid encoding the one or more neoepitopes resulting from the disease-specific mutations of the at least two genes; or (c) administering a peptide or polypeptide comprising the one or more neoepitopes resulting from the disease-specific mutations of the at least two genes. Preferably, all the neoepitopes resulting from the disease-specific mutations of the at least two genes are targeted by the administered immune cells, encoded by the administered nucleic acid, or comprised within the administered peptide or polypeptide.

Moreover, the immune response can be provided to a mammal having a disease, disorder or condition associated with expression of the neoepitope resulting from the disease-specific mutation, such that the disease, disorder or condition is treated or prevented. Preferably, the disease, disorder or condition is cancer.

Preferably, the immune cells are T cells and the antigen receptors are T cell receptors, and the immune response is a T cell-mediated immune response. More preferably, the immune response is an anti-tumor immune response and the target cell population or target tissue expressing the one or more suitable neoepitopes is tumor cells or tumor tissue.

Other features and advantages of the instant invention will be apparent from the following detailed description and claims.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise. For example, if in a preferred embodiment the neoepitope has a high zygosity rather than a high fractional zygosity, and in one preferred embodiment the neoepitope results from a mutation in an essential gene, then in a preferred embodiment, the suitable neoepitope has a high fractional zygosity and results from a mutation in an essential gene, and in a more preferred embodiment the fractional zygosity is equal to 1.

Preferably, the terms used herein are defined as described in "A multilingual glossary of biotechnological terms: (IUPAC Recommendations)", H.G.W. Leuenberger, B. Nagel, and H. Kölbl, Eds., (1995) Helvetica Chimica Acta, CH-4010 Basel, Switzerland.

The practice of the present invention will employ, unless otherwise indicated, conventional methods of biochemistry, cell biology, immunology, and recombinant DNA techniques which are explained in the literature in the field (cf., *e.g.,* Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, *i.e.,* the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. For example, the determination of whether the neoepitope is a suitable disease-specific target by determining the copy number of the encoding gene can be determined before, after or concurrently with the determination that the gene is a driver gene or essential gene, or can be determined before, after or concurrently with the determination that the neoepitope is expressed on the surface of the cell or induces a satisfactory immune response such that it would be suitable in a vaccine.

The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, *etc*.), whether *supra* or *infra,* are hereby incorporated by reference in their entirety. Nothing herein is to be construed as an admission that the invention is not entitled to antedate such disclosure by virtue of prior invention.

The present invention envisions the therapy of diseases, including immunotherapy and radiotherapy, in particular cancer, by targeting neoepitopes ("suitable neoepitopes") that are only expressed in or on the diseased cells and that have the characteristic of being expressed from genes that are less likely to be silenced by the diseased cell, such that the diseased cell is less likely to be able to escape immune surveillance via the targeted neoepitope. The immunotherapy can be effected by active and/or passive immunotherapeutic methods. For example, in one embodiment an antibody or other molecule targeting specifically to a neoepitope and conjugated to a toxic agent capable of killing the cell expressing the neoepitope can be used according to the present invention to target and kill that cell.

The invention specifically is directed to the identification of such suitable neoepitopes as disease-specific targets in immunotherapy. Once suitable neoepitopes have been identified, they can be used in vaccines in order to induce an immune response against the neoepitope, in particular, by inducing and/or activating appropriate effector cells such as T cells that recognize the identified suitable neoepitope, in particular when presented in the context of MHC, via an appropriate antigen receptor, such as a T cell receptor or artificial T cell receptor, which results in the death of the diseased cell expressing the suitable neoepitope. Alternatively, or additionally, immune cells that recognize the identified suitable neoepitope through an appropriate antigen receptor can be administered, which also will result in the death of the cells expressing the suitable neoepitope.

The immunotherapeutic approaches according to the invention include immunization with a peptide or polypeptide containing the suitable neoepitope, ii) nucleic acid encoding the peptide or polypeptide containing the suitable neoepitope, iii) recombinant cells encoding the peptide or polypeptide containing the suitable neoepitope, iv) recombinant viruses encoding the peptide or polypeptide containing the neoepitope and v) antigen presenting cells pulsed with the peptide or polypeptide containing the neoepitope or transfected with nucleic acids encoding the peptide or polypeptide. Other immunotherapeutic approaches according to the invention include transfer of vi) T cell receptors that recognize the neoepitope, and vii) effector cells encoding receptors (such as T cells) that recognize the neoepitope, in particular when presented in the context of MHC.

The term "disease-specific mutation" in the context of the present invention relates to a somatic mutation that is present in the nucleic acid of a diseased cell but absent in the nucleic acid of a corresponding normal, not diseased cell. The disease can be cancer, thus, the term "tumor-specific mutation" or "cancer-specific mutation" relate to a somatic mutation that is present in the nucleic acid of a tumor or cancer cell but absent in the nucleic acid of a corresponding normal, *i.e.* non-tumorous or non-cancerous, cell. The terms "tumor-specific mutation" and "tumor mutation" and the terms "cancer-specific mutation" and "cancer mutation" are used interchangeably herein.

As used herein, a single nucleotide polymorphism (SNP) is a site in the normal genome at which at least one of the two alleles (maternal or paternal) has a different identity from that in the normal genome, or with respect to, for example, a reference genome.

As used herein, a heterozygous single nucleotide polymorphism (heterozygous SNP) is defined as a site in the normal genome at which the two alleles (the maternal and paternal alleles) have a different identity.

As used herein the term "fractional zygosity" refers to the fraction of the copy number of a gene having a disease-specific mutation in view of the total copy number of the gene, whether the gene has the mutation or not. For example, if there are a total of 20 copies of the gene and 10 of the copies have the disease-specific mutation, then the fractional zygosity is 0.5. If all copies of the gene have the disease-specific mutation, then the fractional zygosity is 1. The fractional zygosity can be at least 0.1, 0.2, 0.3, 0.4, 0.5, 0.6, 0.7, 0.8, 0.9, or at least 0.95. In the context of the present invention, a higher fractional zygosity rather than a lower fractional zygosity is preferred. In an embodiment, the fractional zygosity of a mutated allele that encodes an epitope, preferably a neoepitope, is the ratio of the copy number of the mutated allele over the total number of copies of the nucleotide site to which the mutated allele maps, *e.g.,* in a reference genome.

As used herein the term "clonal fraction" refers to the fraction of the number of diseased cells that contain the same disease-specific mutation in the same gene and its genetic characteristics such as copy number and fractional zygosity in view of the total number of diseased cells, whether the diseased cells have the same mutation in the same gene or not. This term can also apply to the tumor tissue in that the clonal fraction is the fraction of diseased cells in the tumor tissue that contain the same disease-specific mutation in the same gene and its genetic characteristics such as copy number in view of the total number of cells in the tumor tissue. For example, in a sample obtained from a tumor, where only half of the total number of tumor cells has the same mutation in the same gene, then the clonal fraction is 0.5. The clonal fraction can be at least 0.1, 0.2, 0.3, 0.4, 0.5, 0.6, 0.7, 0.8, 0.9, or at least 0.95. In the context of the present invention, a higher clonal fraction rather than a lower clonal fraction is preferred. Most preferred is a clonal fraction of 1, in which all of the diseased cells have the same disease-specific mutation in the same gene. "Clonal fraction", "fractional clonality" and "fractional subclonality" are used interchangeably herein.

The term "focal amplification" refers to an amplification, or increase in copy number, of a part of a genome, *e.g.,* amplification of one or more genes located together on the same chromosome, which results in a copy number of greater than 2, preferably greater than 5, 10, 15, 20, 25, 50, 75, 100 for this part of the genome, provided no deletion event for the same part of the genome has occurred. Thus, for the purposes of the present invention, genes that are focally amplified in diseased cells are those genes that can have an increased copy number compared to the wild-type copy number of 2 or the wild-type copy number of 1 for those genes on the X and Y chromosomes in males. Focal amplification is distinct from whole genome duplication and/or amplification events.

The term "immune response" refers to an integrated bodily response to an antigen and preferably refers to a cellular immune response or a cellular as well as a humoral immune response. The immune response may be protective/preventive/prophylactic and/or therapeutic.

"Inducing an immune response" may mean that there was no immune response against a particular antigen before induction, but it may also mean that there was a certain level of immune response against a particular antigen before induction and after induction said immune response is enhanced. Thus, "inducing an immune response" also includes "enhancing an immune response". Preferably, after inducing an immune response in a subject, said subject is protected from developing a disease such as a cancer disease or the disease condition is ameliorated by inducing an immune response. For example, an immune response against a tumor expressed antigen may be induced in a patient having a cancer disease or in a subject being at risk of developing a cancer disease. Inducing an immune response in this case may mean that the disease condition of the subject is ameliorated, that the subject does not develop metastases, or that the subject being at risk of developing a cancer disease does not develop a cancer disease.

A "cellular immune response", a "cellular response", a "cellular response against an antigen" or a similar term is meant to include a cellular response directed to cells characterized by presentation of an antigen with class I or class II MHC. The cellular response relates to cells called T cells or T-lymphocytes which act as either "helpers" or "killers". The helper T cells (also termed CD4⁺ T cells) play a central role by regulating the immune response and the killer cells (also termed cytotoxic T cells, cytolytic T cells, CD8⁺ T cells or CTLs) kill diseased cells such as cancer cells, preventing the production of more diseased cells. Preferably, an anti-tumor CTL response is stimulated against tumor cells expressing one or more tumor expressed antigens and preferably presenting such tumor expressed antigens with class I MHC.

An "antigen" according to the invention covers any substance, preferably a peptide or protein, which is a target of and/or induces an immune response such as a specific reaction with antibodies or T-lymphocytes (T cells). Preferably, an antigen comprises at least one epitope such as a T cell epitope. Preferably, an antigen in the context of the present invention is a molecule which, optionally after processing, induces an immune reaction, which is preferably specific for the antigen (including cells expressing the antigen). The antigen or a T cell epitope thereof is preferably presented by a cell, preferably by an antigen presenting cell which includes a diseased cell, in particular a cancer cell, in the context of MHC molecules, which results in an immune response against the antigen (including cells expressing the antigen).

Preferably, an antigen in the context of the present invention is a molecule which, optionally after processing, induces an immune reaction, which is preferably specific for the antigen. According to the present invention, any suitable antigen may be used, which is a candidate for an immune reaction, wherein the immune reaction may be both a humoral as well as a cellular immune reaction. In the context of the present invention, the antigen is preferably presented by a cell, preferably by an antigen presenting cell, in the context of MHC molecules, which results in an immune reaction against the antigen. An antigen is preferably a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. According to the present invention, an antigen may correspond to a naturally occurring product, for example, a viral protein, or a part thereof. In preferred embodiments, the antigen is a surface polypeptide, *i.e.,* a polypeptide naturally displayed on the surface of a cell, a pathogen, a bacterium, a virus, a fungus, a parasite, an allergen, or a tumor. The antigen may elicit an immune response against a cell, a pathogen, a bacterium, a virus, a fungus, a parasite, an allergen, or a tumor.

The term "disease-associated antigen" or "disease-specific antigen" is used in it broadest sense to refer to any antigen associated with or specific to a disease. Such an antigen is a molecule which contains epitopes that will stimulate a host's immune system to make a cellular antigen-specific immune response and/or a humoral antibody response against the disease. The disease-associated antigen may therefore be used for therapeutic purposes. Disease-associated antigens are preferably associated with infection by microbes, typically microbial antigens, or associated with cancer, typically tumors.

The term "pathogen" refers to pathogenic biological material capable of causing disease in an organism, preferably a vertebrate organism. Pathogens include microorganisms such as bacteria, unicellular eukaryotic organisms (protozoa), fungi, as well as viruses.

In the context of the present invention, the term "tumor antigen" or "tumor-associated antigen" relates to proteins that are under normal conditions specifically expressed in a limited number of tissues and/or organs or in specific developmental stages, for example, the tumor antigen may be under normal conditions specifically expressed in stomach tissue, preferably in the gastric mucosa, in reproductive organs, *e.g.,* in testis, in trophoblastic tissue, *e.g.,* in placenta, or in germ line cells, and are expressed or aberrantly expressed in one or more tumor or cancer tissues. In this context, "a limited number" preferably means not more than 3, more preferably not more than 2. The tumor antigens in the context of the present invention include, for example, differentiation antigens, preferably cell type specific differentiation antigens, *i.e.,* proteins that are under normal conditions specifically expressed in a certain cell type at a certain differentiation stage, cancer/testis antigens, *i.e.,* proteins that are under normal conditions specifically expressed in testis and sometimes in placenta, and germ line specific antigens. In the context of the present invention, the tumor antigen is preferably associated with the cell surface of a cancer cell and is preferably not or only rarely expressed in normal tissues. Preferably, the tumor antigen or the aberrant expression of the tumor antigen identifies cancer cells. In the context of the present invention, the tumor antigen that is expressed by a cancer cell in a subject, e.g., a patient suffering from a cancer disease, is preferably a self-protein in said subject. In preferred embodiments, the tumor antigen in the context of the present invention is expressed under normal conditions specifically in a tissue or organ that is non-essential, *i.e.,* tissues or organs which when damaged by the immune system do not lead to death of the subject, or in organs or structures of the body which are not or only hardly accessible by the immune system. Preferably, the amino acid sequence of the tumor antigen is identical between the tumor antigen which is expressed in normal tissues and the tumor antigen which is expressed in cancer tissues.

According to the invention, the terms "tumor antigen", "tumor-expressed antigen", "cancer antigen" and "cancer-expressed antigen" are equivalents and are used interchangeably herein.

The terms "epitope", "antigen peptide", "antigen epitope", "immunogenic peptide" and "MHC binding peptide" are used interchangeably herein and refer to an antigenic determinant in a molecule such as an antigen, *i.e.,* to a part in or fragment of an immunologically active compound that is recognized by the immune system, for example, that is recognized by a T cell, in particular when presented in the context of MHC molecules. An epitope of a protein preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 9, 10, 11, 12, 13, 14, 15, 16, 17, 18, 19, 20, 21, 22, 23, 24, or 25 amino acids in length. According to the invention an epitope may bind to MHC molecules such as MHC molecules on the surface of a cell and thus, may be a "MHC binding peptide" or "antigen peptide". The term "major histocompatibility complex" and the abbreviation "MHC" include MHC class I and MHC class II molecules and relate to a complex of genes which is present in all vertebrates. MHC proteins or molecules are important for signaling between lymphocytes and antigen presenting cells or diseased cells in immune reactions, wherein the MHC proteins or molecules bind peptides and present them for recognition by T cell receptors. The proteins encoded by the MHC are expressed on the surface of cells, and display both self-antigens (peptide fragments from the cell itself) and non-self-antigens (e.g., fragments of invading microorganisms) to a T cell. Preferred such immunogenic portions bind to an MHC class I or class II molecule. As used herein, an immunogenic portion is said to "bind to" an MHC class I or class II molecule if such binding is detectable using any assay known in the art. The term "MHC binding peptide" relates to a peptide which binds to an MHC class I and/or an MHC class II molecule. In the case of class I MHC/peptide complexes, the binding peptides are typically 8-10 amino acids long although longer or shorter peptides may be effective. In the case of class II MHC/peptide complexes, the binding peptides are typically 10-25 amino acids long and are in particular 13-18 amino acids long, whereas longer and shorter peptides may be effective.

As used herein the term "neoepitope" refers to an epitope that is not present in a reference such as a normal non-cancerous or germline cell but is found in diseased cells, such as cancer cells. This includes, in particular, situations wherein in a normal non-cancerous or germline cell a corresponding epitope is found, however, due to one or more mutations in a cancer cell the sequence of the epitope is changed so as to result in the neoepitope. Moreover, a neoepitope may not only be specific to the diseased cells but also can be specific to the patient having the disease. Since neoepitopes and suitable neoepitopes which are identified by the methods of the invention are subsets of epitopes, disclosure herein relating to epitopes in general as immunological targets applies equally to neoepitopes and suitable neoepitopes.

In one particularly preferred embodiment of the invention, an epitope or neoepitope is a T cell epitope. As used herein, the term "T cell epitope" refers to a peptide which binds to a MHC molecule in a configuration recognized by a T cell receptor. Typically, T cell epitopes are presented on the surface of an antigen-presenting cell.

As used herein, the term "predicting immunogenic amino acid modifications" refers to a prediction whether a peptide comprising such amino acid modification will be immunogenic and thus useful as epitope, in particular T cell epitope, in vaccination.

According to the invention, a T cell epitope may be present in a vaccine as a part of a larger entity such as a vaccine sequence and/or a polypeptide comprising more than one T cell epitope. The presented peptide or T cell epitope is produced following suitable processing.

T cell epitopes may be modified at one or more residues that are not essential for TCR recognition or for binding to MHC. Such modified T cell epitopes may be considered immunologically equivalent.

Preferably a T cell epitope when presented by MHC and recognized by a T cell receptor is able to induce in the presence of appropriate co-stimulatory signals, clonal expansion of the T cell carrying the T cell receptor specifically recognizing the peptide/MHC-complex.

Preferably, a T cell epitope comprises an amino acid sequence substantially corresponding to the amino acid sequence of a fragment of an antigen. Preferably, said fragment of an antigen is an MHC class I and/or class II presented peptide.

A T cell epitope according to the invention preferably relates to a portion or fragment of an antigen which is capable of stimulating an immune response, preferably a cellular response against the antigen or cells characterized by expression of the antigen and preferably by presentation of the antigen such as diseased cells, in particular cancer cells. Preferably, a T cell epitope is capable of stimulating a cellular response against a cell characterized by presentation of an antigen with class I MHC and preferably is capable of stimulating an antigen-responsive cytotoxic T-lymphocyte (CTL).

In some embodiments the antigen is a self-antigen, particularly a tumor antigen. Tumor antigens and their determination are known to the skilled person.

The term "immunogenicity" relates to the relative effectivity to induce an immune response that is preferably associated with therapeutic treatments, such as treatments against cancers. As used herein, the term "immunogenic" relates to the property of having immunogenicity. For example, the term "immunogenic modification" when used in the context of a peptide, polypeptide or protein relates to the effectivity of said peptide, polypeptide or protein to induce an immune response that is caused by and/or directed against said modification. Preferably, the non-modified peptide, polypeptide or protein does not induce an immune response, induces a different immune response or induces a different level, preferably a lower level, of immune response.

According to the invention, the term "immunogenicity" or "immunogenic" preferably relates to the relative effectivity to induce a biologically relevant immune response, in particular an immune response which is useful for vaccination. Thus, in one preferred embodiment, an amino acid modification or modified peptide is immunogenic if it induces an immune response against the target modification in a subject, which immune response may be beneficial for therapeutic or prophylactic purposes.

"Antigen processing" or "processing" refers to the degradation of a polypeptide or antigen into procession products, which are fragments of said polypeptide or antigen (e.g., the degradation of a polypeptide into peptides) and the association of one or more of these fragments (e.g., via binding) with MHC molecules for presentation by cells, preferably antigen presenting cells, to specific T cells.

"Antigen presenting cells" (APC) are cells which present peptide fragments of protein antigens in association with MHC molecules on their cell surface. Some APCs may activate antigen specific T cells.

Professional antigen-presenting cells are very efficient at internalizing antigen, either by phagocytosis or by receptor-mediated endocytosis, and then displaying a fragment of the antigen, bound to a class II MHC molecule, on their membrane. The T cell recognizes and interacts with the antigen-class II MHC molecule complex on the membrane of the antigen-presenting cell. An additional co-stimulatory signal is then produced by the antigen-presenting cell, leading to activation of the T cell. The expression of co-stimulatory molecules is a defining feature of professional antigen-presenting cells.

The main types of professional antigen-presenting cells are dendritic cells, which have the broadest range of antigen presentation, and are probably the most important antigen-presenting cells, macrophages, B-cells, and certain activated epithelial cells.

Dendritic cells (DCs) are leukocyte populations that present antigens captured in peripheral tissues to T cells via both MHC class II and I antigen presentation pathways. It is well known that dendritic cells are potent inducers of immune responses and the activation of these cells is a critical step for the induction of anti-tumor immunity. Dendritic cells are conveniently categorized as "immature" and "mature" cells, which can be used as a simple way to discriminate between two well characterized phenotypes. However, this nomenclature should not be construed to exclude all possible intermediate stages of differentiation. Immature dendritic cells are characterized as antigen presenting cells with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g.,* CD54 and CD11) and costimulatory molecules (*e.g.,* CD40, CD80, CD86 and 4-1 BB).

Dendritic cell maturation is referred to as the status of dendritic cell activation at which such antigen-presenting dendritic cells lead to T cell priming, while presentation by immature dendritic cells results in tolerance. Dendritic cell maturation is chiefly caused by biomolecules with microbial features detected by innate receptors (bacterial DNA, viral RNA, endotoxin, etc.), pro-inflammatory cytokines (TNF, IL-1, IFNs), ligation of CD40 on the dendritic cell surface by CD40L, and substances released from cells undergoing stressful cell death. The dendritic cells can be derived by culturing bone marrow cells *in vitro* with cytokines, such as granulocyte-macrophage colony-stimulating factor (GM-CSF) and tumor necrosis factor alpha.

Non-professional antigen-presenting cells do not constitutively express the MHC class II proteins required for interaction with naive T cells; these are expressed only upon stimulation of the non-professional antigen-presenting cells by certain cytokines such as IFNγ.

"Antigen presenting cells" can be loaded with MHC class I presented peptides by transducing the cells with nucleic acid, preferably RNA, encoding a peptide or polypeptide comprising the peptide to be presented, *e.g.* a nucleic acid encoding the antigen.

In some embodiments, a pharmaceutical composition of the invention comprising a gene delivery vehicle that targets a dendritic or other antigen presenting cell may be administered to a patient, resulting in transfection that occurs *in vivo. In vivo* transfection of dendritic cells, for example, may generally be performed using any methods known in the art, such as those described in WO 97/24447, or the gene gun approach described by Mahvi et al., Immunology and cell Biology 75:456-460, 1997.

The term "antigen presenting cell" also includes target cells.

"Target cell" shall mean a cell which is a target for an immune response such as a cellular immune response. Target cells include cells that present an antigen or an antigen epitope, *i.e.* a peptide fragment derived from an antigen, and include any undesirable cell such as a cancer cell. In preferred embodiments, the target cell is a cell expressing an antigen as described herein and preferably presenting said antigen with class I MHC.

The term "portion" refers to a fraction. With respect to a particular structure such as an amino acid sequence or protein the term "portion" thereof may designate a continuous or a discontinuous fraction of said structure. Preferably, a portion of an amino acid sequence comprises at least 1%, at least 5%, at least 10%, at least 20%, at least 30%, preferably at least 40%, preferably at least 50%, more preferably at least 60%, more preferably at least 70%, even more preferably at least 80%, and most preferably at least 90% of the amino acids of said amino acid sequence. Preferably, if the portion is a discontinuous fraction said discontinuous fraction is composed of 2, 3, 4, 5, 6, 7, 8, or more parts of a structure, each part being a continuous element of the structure. For example, a discontinuous fraction of an amino acid sequence may be composed of 2, 3, 4, 5, 6, 7, 8, or more, preferably not more than 4 parts of said amino acid sequence, wherein each part preferably comprises at least 5 continuous amino acids, at least 10 continuous amino acids, preferably at least 20 continuous amino acids, preferably at least 30 continuous amino acids of the amino acid sequence.

The terms "part" and "fragment" are used interchangeably herein and refer to a continuous element. For example, a part of a structure such as an amino acid sequence or protein refers to a continuous element of said structure. A portion, a part or a fragment of a structure preferably comprises one or more functional properties of said structure. For example, a portion, a part or a fragment of an epitope, peptide or protein is preferably immunologically equivalent to the epitope, peptide or protein it is derived from. In the context of the present invention, a "part" of a structure such as an amino acid sequence preferably comprises, preferably consists of at least 10%, at least 20%, at least 30%, at least 40%, at least 50%, at least 60%, at least 70%, at least 80%, at least 85%, at least 90%, at least 92%, at least 94%, at least 96%, at least 98%, at least 99% of the entire structure or amino acid sequence.

The term "immunoreactive cell" in the context of the present invention relates to a cell which exerts effector functions during an immune reaction. An "immunoreactive cell" preferably is capable of binding an antigen or a cell characterized by presentation of an antigen or an antigen peptide derived from an antigen and mediating an immune response. For example, such cells secrete cytokines and/or chemokines, secrete antibodies, recognize cancerous cells, and optionally eliminate such cells. For example, immunoreactive cells comprise T cells (cytotoxic T cells, helper T cells, tumor infiltrating T cells), B cells, natural killer cells, neutrophils, macrophages, and dendritic cells. Preferably, in the context of the present invention, "immunoreactive cells" are T cells, preferably CD4⁺ and/or CD8⁺ T cells.

Preferably, an "immunoreactive cell" recognizes an antigen or an antigen peptide derived from an antigen with some degree of specificity, in particular if presented in the context of MHC molecules such as on the surface of antigen presenting cells or diseased cells such as cancer cells. Preferably, said recognition enables the cell that recognizes an antigen or an antigen peptide derived from said antigen to be responsive or reactive. If the cell is a helper T cell (CD4⁺ T cell) bearing receptors that recognize an antigen or an antigen peptide derived from an antigen in the context of MHC class II molecules such responsiveness or reactivity may involve the release of cytokines and/or the activation of CD8⁺ lymphocytes (CTLs) and/or B-cells. If the cell is a CTL such responsiveness or reactivity may involve the elimination of cells presented in the context of MHC class I molecules, *i.e.,* cells characterized by presentation of an antigen with class I MHC, for example, via apoptosis or perforin-mediated cell lysis. CTL responsiveness may include sustained calcium flux, cell division, production of cytokines such as IFN-γ and TNF-α, up-regulation of activation markers such as CD44 and CD69, and specific cytolytic killing of antigen expressing target cells. CTL responsiveness may also be determined using an artificial reporter that accurately indicates CTL responsiveness. Such CTL that recognizes an antigen or an antigen peptide derived from an antigen and are responsive or reactive are also termed "antigen-responsive CTL" herein. If the cell is a B cell such responsiveness may involve the release of immunoglobulins.

The terms "T cell" and "T lymphocyte" are used interchangeably herein and include T helper cells (CD4+ T cells) and cytotoxic T cells (CTLs, CD8+ T cells) which comprise cytolytic T cells.

T cells belong to a group of white blood cells known as lymphocytes, and play a central role in cell-mediated immunity. They can be distinguished from other lymphocyte types, such as B cells and natural killer cells by the presence of a special receptor on their cell surface called T cell receptor (TCR). The thymus is the principal organ responsible for the maturation of T cells. Several different subsets of T cells have been discovered, each with a distinct function.

T helper cells assist other white blood cells in immunologic processes, including maturation of B cells into plasma cells and activation of cytotoxic T cells and macrophages, among other functions. These cells are also known as CD4+ T cells because they express the CD4 protein on their surface. Helper T cells become activated when they are presented with peptide antigens by MHC class II molecules that are expressed on the surface of antigen presenting cells (APCs). Once activated, they divide rapidly and secrete small proteins called cytokines that regulate or assist in the active immune response.

Cytotoxic T cells destroy virally infected cells and tumor cells, and are also implicated in transplant rejection. These cells are also known as CD8+ T cells since they express the CD8 glycoprotein at their surface. These cells recognize their targets by binding to antigen associated with MHC class I, which is present on the surface of nearly every cell of the body.

A majority of T cells have a T cell receptor (TCR) existing as a complex of several proteins. The actual T cell receptor is composed of two separate peptide chains, which are produced from the independent T cell receptor alpha and beta (TCRα and TCRβ genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells.

According to the invention, the term "antigen receptor" includes naturally occurring receptors such as T cell receptor as well as engineered receptors, which confer an arbitrary specificity such as the specificity of a monoclonal antibody onto an immune effector cell such as a T cell. In this way, a large number of antigen-specific T cells can be generated for adoptive cell transfer. Thus, an antigen receptor according to the invention may be present on T cells, *e.g.* instead of or in addition to the T cell's own T cell receptor. Such T cells do not necessarily require processing and presentation of an antigen for recognition of the target cell but rather may recognize preferably with specificity any antigen present on a target cell. Preferably, said antigen receptor is expressed on the surface of the cells. For the purpose of the present invention, T cells comprising an antigen receptor are comprised by the term "T cell" as used herein. Specifically, according to the invention, the term "antigen receptor" includes artificial receptors comprising a single molecule or a complex of molecules which recognize, *i.e.* bind to, a target structure (*e.g.* an antigen) on a target cell such as a cancer cell (*e.g.* by binding of an antigen binding site or antigen binding domain to an antigen expressed on the surface of the target cell) and may confer specificity onto an immune effector cell such as a T cell expressing said antigen receptor on the cell surface. Preferably, recognition of the target structure by an antigen receptor results in activation of an immune effector cell expressing said antigen receptor. An antigen receptor may comprise one or more protein units said protein units comprising one or more domains as described herein. According to the invention an "antigen receptor" also may be a "chimeric antigen receptor (CAR)", "chimeric T cell receptor" or "artificial T cell receptor".

An antigen can be recognized by an antigen receptor through any antigen recognition domains (herein also referred to simply as "domains") able to form an antigen binding site such as through antigen-binding portions of antibodies and T cell receptors which may reside on the same or different peptide chains. In one embodiment, the two domains forming an antigen binding site are derived from an immunoglobulin. In one embodiment, the two domains forming an antigen binding site are derived from a T cell receptor. Particularly preferred are antibody variable domains, such as single-chain variable fragments (scFv) derived from monoclonal antibodies and T cell receptor variable domains, in particular TCR alpha and beta single chains. In fact almost anything that binds a given target with high affinity can be used as an antigen recognition domain.

The first signal in activation of T cells is provided by binding of the T cell receptor to a short peptide presented by the major histocompatibility complex (MHC) on another cell. This ensures that only a T cell with a TCR specific to that peptide is activated. The partner cell is usually a professional antigen presenting cell (APC), usually a dendritic cell in the case of naive responses, although B cells and macrophages can be important APCs. The peptides presented to CD8+ T cells by MHC class I molecules are typically 8-10 amino acids in length; the peptides presented to CD4+ T cells by MHC class II molecules are typically longer, as the ends of the binding cleft of the MHC class II molecule are open.

According to the present invention, a molecule is capable of binding to a target if it has a significant affinity for said predetermined target and binds to said predetermined target in standard assays. "Affinity" or "binding affinity" is often measured by equilibrium dissociation constant (K_{D}). A molecule is not (substantially) capable of binding to a target if it has no significant affinity for said target and does not bind significantly to said target in standard assays.

Cytotoxic T lymphocytes may be generated *in vivo* by incorporation of an antigen or an antigen peptide into antigen-presenting cells *in vivo.* The antigen or antigen peptide may be represented as protein, as DNA (e.g. within a vector) or as RNA. The antigen may be processed to produce a peptide partner for the MHC molecule, while a fragment thereof may be presented without the need for further processing. The latter is the case in particular, if these can bind to MHC molecules. In general, administration to a patient by intradermal injection is possible. However, injection may also be carried out intranodally into a lymph node (Maloy et al., 2001, Proc Natl Acad Sci USA 98:3299-303). The resulting cells present the complex of interest and are recognized by autologous cytotoxic T lymphocytes which then propagate.

Specific activation of CD4+ or CD8+ T cells may be detected in a variety of ways. Methods for detecting specific T cell activation include detecting the proliferation of T cells, the production of cytokines (e.g., lymphokines), or the generation of cytolytic activity. For CD4+ T cells, a preferred method for detecting specific T cell activation is the detection of the proliferation of T cells. For CD8+ T cells, a preferred method for detecting specific T cell activation is the detection of the generation of cytolytic activity.

By "cell characterized by presentation of an antigen" or "cell presenting an antigen" or similar expressions is meant a cell such as a diseased cell, *e.g.* a cancer cell, or an antigen presenting cell presenting the antigen it expresses or a fragment derived from said antigen, e.g. by processing of the antigen, in the context of MHC molecules, in particular MHC Class I molecules. Similarly, the terms "disease characterized by presentation of an antigen" denotes a disease involving cells characterized by presentation of an antigen, in particular with class I MHC. Presentation of an antigen by a cell may be effected by transfecting the cell with a nucleic acid such as RNA encoding the antigen.

By "fragment of an antigen which is presented" or similar expressions is meant that the fragment can be presented by MHC class I or class II, preferably MHC class I, *e.g.* when added directly to antigen presenting cells. In one embodiment, the fragment is a fragment which is naturally presented by cells expressing an antigen.

The term "immunologically equivalent" means that the immunologically equivalent molecule such as the immunologically equivalent amino acid sequence exhibits the same or essentially the same immunological properties and/or exerts the same or essentially the same immunological effects, *e.g.,* with respect to the type of the immunological effect such as induction of a humoral and/or cellular immune response, the strength and/or duration of the induced immune reaction, or the specificity of the induced immune reaction. In the context of the present invention, the term "immunologically equivalent" is preferably used with respect to the immunological effects or properties of a peptide used for immunization. For example, an amino acid sequence is immunologically equivalent to a reference amino acid sequence if said amino acid sequence when exposed to the immune system of a subject induces an immune reaction having a specificity of reacting with the reference amino acid sequence.

The term "immune effector functions" in the context of the present invention includes any functions mediated by components of the immune system that result, for example, in the killing of tumor cells, or in the inhibition of tumor growth and/or inhibition of tumor development, including inhibition of tumor dissemination and metastasis. Preferably, the immune effector functions in the context of the present invention are T cell mediated effector functions. Such functions comprise in the case of a helper T cell (CD4+ T cell) the recognition of an antigen or an antigen peptide derived from an antigen in the context of MHC class II molecules by T cell receptors, the release of cytokines and/or the activation of CD8+ lymphocytes (CTLs) and/or B-cells, and in the case of CTL the recognition of an antigen or an antigen peptide derived from an antigen in the context of MHC class I molecules by T cell receptors, the elimination of cells presented in the context of MHC class I molecules, *i.e.,* cells characterized by presentation of an antigen with class I MHC, for example, via apoptosis or perforin-mediated cell lysis, production of cytokines such as IFN-γ and TNF-α, and specific cytolytic killing of antigen expressing target cells.

The terms "major histocompatibility complex" and the abbreviation "MHC" include MHC class I and MHC class II molecules and relate to a complex of genes which occurs in all vertebrates. MHC proteins or molecules are important for signaling between lymphocytes and antigen presenting cells or diseased cells in immune reactions, wherein the MHC proteins or molecules bind peptides and present them for recognition by T cell receptors. The proteins encoded by the MHC are expressed on the surface of cells, and display both self antigens (peptide fragments from the cell itself) and non-self antigens (e.g., fragments of invading microorganisms) to a T cell.

The MHC region is divided into three subgroups, class I, class II, and class III. MHC class I proteins contain an α-chain and β2-microglobulin (not part of the MHC encoded by chromosome 15). They present antigen fragments to cytotoxic T cells. On most immune system cells, specifically on antigen-presenting cells, MHC class II proteins contain α- and β-chains and they present antigen fragments to T-helper cells. MHC class III region encodes for other immune components, such as complement components and some that encode cytokines.

The MHC is both polygenic (there are several MHC class I and MHC class II genes) and polymorphic (there are multiple alleles of each gene).

As used herein, the term "haplotype" refers to the HLA alleles found on one chromosome and the proteins encoded thereby. Haplotype may also refer to the allele present at any one locus within the MHC. Each class of MHC is represented by several loci: e.g., HLA-A (Human Leukocyte Antigen-A), HLA-B, HLA-C, HLA-E, HLA-F, HLA-G, HLA-H, HLA-J, HLA-K, HLA-L, HLA-P and HLA-V for class I and HLA-DRA, HLA-DRB1-9, HLA-DQA1, HLA-DQB1, HLA-DPA1, HLA-DPB1, HLA-DMA, HLA-DMB, HLA-DOA, and HLA-DOB for class II. The terms "HLA allele" and "MHC allele" are used interchangeably herein.

The MHCs exhibit extreme polymorphism. Within the human population there are, at each genetic locus, a great number of haplotypes comprising distinct alleles. Different polymorphic MHC alleles, of both class I and class II, have different peptide specificities in that each allele encodes proteins that bind peptides exhibiting particular sequence patterns.

In the context of the present invention, a MHC molecule is preferably an HLA molecule.

In the context of the present invention, the term "MHC binding peptide" includes MHC class I and/or class II binding peptides or peptides that can be processed to produce MHC class I and/or class II binding peptides. In the case of class I MHC/peptide complexes, the binding peptides are typically 8-12, preferably 8-10 amino acids long although longer or shorter peptides may be effective. In the case of class II MHC/peptide complexes, the binding peptides are typically 9-30, preferably 10-25 amino acids long and are in particular 13-18 amino acids long, whereas longer and shorter peptides may be effective.

An "antigen peptide" preferably relates to a portion or fragment of an antigen which is capable of stimulating an immune response, preferably a cellular response against the antigen or cells characterized by expression of the antigen and preferably by presentation of the antigen such as diseased cells, in particular cancer cells. Preferably, an antigen peptide is capable of stimulating a cellular response against a cell characterized by presentation of an antigen with class I MHC and preferably is capable of stimulating an antigen-responsive cytotoxic T-lymphocyte (CTL). Preferably, the antigen peptides are MHC class I and/or class II presented peptides or can be processed to produce MHC class I and/or class II presented peptides. Preferably, the antigen peptides comprise an amino acid sequence substantially corresponding to the amino acid sequence of a fragment of an antigen. Preferably, said fragment of an antigen is an MHC class I and/or class II presented peptide. Preferably, an antigen peptide comprises an amino acid sequence substantially corresponding to the amino acid sequence of such fragment and is processed to produce such fragment, *i.e.,* an MHC class I and/or class II presented peptide derived from an antigen.

If a peptide is to be presented directly, *i.e.,* without processing, in particular without cleavage, it has a length which is suitable for binding to an MHC molecule, in particular a class I MHC molecule, and preferably is 7-20 amino acids in length, more preferably 7-12 amino acids in length, more preferably 8-11 amino acids in length, in particular 9 or 10 amino acids in length.

If a peptide is part of a larger entity comprising additional sequences, *e.g.* of a vaccine sequence or polypeptide, and is to be presented following processing, in particular following cleavage, the peptide produced by processing has a length which is suitable for binding to an MHC molecule, in particular a class I MHC molecule, and preferably is 7-20 amino acids in length, more preferably 7-12 amino acids in length, more preferably 8-11 amino acids in length, in particular 9 or 10 amino acids in length. Preferably, the sequence of the peptide which is to be presented following processing is derived from the amino acid sequence of an antigen, *i.e.,* its sequence substantially corresponds and is preferably completely identical to a fragment of an antigen. Thus, an MHC binding peptide comprises a sequence which substantially corresponds and is preferably completely identical to a fragment of an antigen.

Peptides having amino acid sequences substantially corresponding to a sequence of a peptide which is presented by the class I MHC may differ at one or more residues that are not essential for TCR recognition of the peptide as presented by the class I MHC, or for peptide binding to MHC. Such substantially corresponding peptides are also capable of stimulating an antigen-responsive CTL and may be considered immunologically equivalent. Peptides having amino acid sequences differing from a presented peptide at residues that do not affect TCR recognition but improve the stability of binding to MHC may improve the immunogenicity of the antigen peptide, and may be referred to herein as "optimized peptide". Using existing knowledge about which of these residues may be more likely to affect binding either to the MHC or to the TCR, a rational approach to the design of substantially corresponding peptides may be employed. Resulting peptides that are functional are contemplated as antigen peptides.

An antigen peptide when presented by MHC should be recognizable by a T cell receptor. Preferably, the antigen peptide if recognized by a T cell receptor is able to induce in the presence of appropriate co-stimulatory signals, clonal expansion of the T cell carrying the T cell receptor specifically recognizing the antigen peptide. Preferably, antigen peptides, in particular if presented in the context of MHC molecules, are capable of stimulating an immune response, preferably a cellular response against the antigen from which they are derived or cells characterized by expression of the antigen and preferably characterized by presentation of the antigen. Preferably, an antigen peptide is capable of stimulating a cellular response against a cell characterized by presentation of the antigen with class I MHC and preferably is capable of stimulating an antigen-responsive CTL. Such cell preferably is a target cell.

The term "genome" relates to the total amount of genetic information in the chromosomes of an organism or a cell.

The term "exome" refers to part of the genome of an organism formed by exons, which are coding portions of expressed genes. The exome provides the genetic blueprint used in the synthesis of proteins and other functional gene products. It is the most functionally relevant part of the genome and, therefore, it is most likely to contribute to the phenotype of an organism. The exome of the human genome is estimated to comprise 1.5% of the total genome (Ng et al., 2008, PLoS Gen., 4(8):1-15).

The term "transcriptome" relates to the set of all RNA molecules, including mRNA, rRNA, tRNA, and other non-coding RNA produced in one cell or a population of cells. In context of the present invention the transcriptome or RNAseq means the set of all RNA molecules produced in one cell, a population of cells, preferably a population of cancer cells, or all cells of a given individual at a certain time point.

A "nucleic acid" is preferably deoxyribonucleic acid (DNA) or ribonucleic acid (RNA), more preferably RNA, most preferably *in vitro* transcribed RNA (IVT RNA) or synthetic RNA. Nucleic acids include genomic DNA, cDNA, mRNA, recombinantly produced and chemically synthesized molecules. A nucleic acid may be present as a single-stranded or double-stranded and linear or covalently circularly closed molecule. A nucleic acid can be isolated. The term "isolated nucleic acid" means that the nucleic acid (i) was amplified *in vitro,* for example via polymerase chain reaction (PCR), (ii) was produced recombinantly by cloning, (iii) was purified, for example, by cleavage and separation by gel electrophoresis, or (iv) was synthesized, for example, by chemical synthesis. A nucleic can be employed for introduction into, *i.e.* transfection of, cells, in particular, in the form of RNA which can be prepared by *in vitro* transcription from a DNA template. The RNA can moreover be modified before application by stabilizing sequences, capping, and polyadenylation.

The term "genetic material" refers to isolated nucleic acid, either DNA or RNA, a section of a double helix, a section of a chromosome, or an organism's or cell's entire genome, in particular its exome or transcriptome.

The term "mutation" refers to a change of or difference in the nucleic acid sequence (nucleotide substitution, addition or deletion) in the diseased genome compared to a reference, and preferably a matched normal genome. A "somatic mutation" can occur in any of the cells of the body except the germ cells (sperm and egg) and therefore are not passed on to children. These alterations can (but do not always) cause cancer or other diseases. Preferably a mutation is a non-synonymous mutation. The term "non-synonymous mutation" refers to a mutation, preferably a nucleotide substitution, which results in an amino acid change such as an amino acid substitution in the translation product, which preferably results in the formation of a neoepitope.

The term "single nucleotide variant/variation" (SNV) refers to a difference in the nucleic acid sequence at a particular site (allele) when comparing a genome from a diseased cell, such as a tumor cell, and a genome of a preferably matched (corresponding) normal, non-diseased cell or a reference genome. As used herein, the term mutation preferably encompasses a SNV.

A copy number variation (CNV) event in the diseased (tumor) genome is a somatic copy number variation event occurring only in diseased cells, and defined as a change in the number of copies of the maternal and/or paternal alleles of a region of the diseased (tumor) genome with respect to a matched normal genome, where the alternation preferably affects a region of the genome spanning approximately 1 kb or longer.

The term "mutation" includes point mutations, indels, fusions, chromothripsis and RNA edits.

The term "indel" describes a special mutation class, defined as a mutation resulting in a colocalized insertion and deletion and a net gain or loss in nucleotides. In coding regions of the genome, unless the length of an indel is a multiple of 3, they produce a frameshift mutation. Indels can be contrasted with a point mutation; where an Indel inserts and deletes nucleotides from a sequence, a point mutation is a form of substitution that replaces one of the nucleotides.

Fusions can generate hybrid genes formed from two previously separate genes. It can occur as the result of a translocation, interstitial deletion, or chromosomal inversion. Often, fusion genes are oncogenes. Oncogenic fusion genes may lead to a gene product with a new or different function from the two fusion partners. Alternatively, a proto-oncogene is fused to a strong promoter, and thereby the oncogenic function is set to function by an upregulation caused by the strong promoter of the upstream fusion partner. Oncogenic fusion transcripts may also be caused by trans-splicing or read-through events.

The term "chromothripsis" refers to a genetic phenomenon by which specific regions of the genome are shattered and then stitched together via a single devastating event.

The term "RNA edit" or "RNA editing" refers to molecular processes in which the information content in an RNA molecule is altered through a chemical change in the base makeup. RNA editing includes nucleoside modifications such as cytidine (C) to uridine (U) and adenosine (A) to inosine (I) deaminations, as well as non-templated nucleotide additions and insertions. RNA editing in mRNAs effectively alters the amino acid sequence of the encoded protein so that it differs from that predicted by the genomic DNA sequence.

The term "cancer mutation signature" refers to a set of mutations which are present in cancer cells when compared to non-cancerous reference cells.

A "reference" in the context of the present invention may be used to correlate and compare the results obtained from a tumor specimen. Typically the "reference" may be obtained on the basis of one or more normal specimens, in particular specimens which are not affected by a cancer disease, either obtained from a patient or one or more different individuals, preferably healthy individuals, in particular individuals of the same species. A "reference" can be determined empirically by testing a sufficiently large number of normal specimens.

The term "reference genome" refers to a genome providing a coordinate system for the normal genome and the diseased genome. A reference genome is used for mapping reads and providing a coordinate system for the normal genome and the tumor genome, wherein the coordinate system allows for the provision of the chromosome number, a nucleotide position in the chromosome, as well as directionality of the read. A reference genome can be based on the genome of one or more members from the same species as the subject providing the diseased sample, or can be based on the normal genome of the subject (a matched genome).

Any suitable sequencing method can be used in the context of the present invention to identify disease-specific mutations, Next Generation Sequencing (NGS) technologies being preferred, and optionally in combination with SNP arrays to obtain absolute copy number information. Third Generation Sequencing methods might substitute for the NGS technology in the future to speed up the sequencing step of the method. For clarification purposes: the terms "Next Generation Sequencing" or "NGS" in the context of the present invention mean all novel high throughput sequencing technologies which, in contrast to the "conventional" sequencing methodology known as Sanger chemistry, read nucleic acid templates randomly in parallel along the entire genome by breaking the entire genome into small pieces. Such NGS technologies (also known as massively parallel sequencing technologies) are able to deliver nucleic acid sequence information of a whole genome, exome, transcriptome (all transcribed sequences of a genome) or methylome (all methylated sequences of a genome) in very short time periods, *e.g.* within 1-2 weeks, preferably within 1-7 days or most preferably within less than 24 hours and allow, in principle, single cell sequencing approaches. Multiple NGS platforms which are commercially available or which are mentioned in the literature can be used in the context of the present invention *e.g.* those described in detail in Zhang et al., 2011, The impact of next-generation sequencing on genomics, J. Genet Genomics 38(3):95-109; or in Voelkerding et al., 2009, Next generation sequencing: From basic research to diagnostics, Clinical chemistry 55:641-658. Non-limiting examples of such NGS technologies/platforms are
1) The sequencing-by-synthesis technology known as pyrosequencing implemented e.g. in the GS-FLX 454 Genome Sequencer^{™} of Roche-associated company 454 Life Sciences (Branford, Connecticut), first described in Ronaghi et al., 1998, A sequencing method based on real-time pyrophosphate, Science 281:363-365. This technology uses an emulsion PCR in which single-stranded DNA binding beads are encapsulated by vigorous vortexing into aqueous micelles containing PCR reactants surrounded by oil for emulsion PCR amplification. During the pyrosequencing process, light emitted from phosphate molecules during nucleotide incorporation is recorded as the polymerase synthesizes the DNA strand.
2) The sequencing-by-synthesis approaches developed by Solexa (now part of Illumina Inc., San Diego, California) which is based on reversible dye-terminators and implemented *e.g.* in the Illumina/Solexa Genome Analyzer^{™} and in the Illumina HiSeq 2000 Genome Analyzer^{™}. In this technology, all four nucleotides are added simultaneously into oligo-primed cluster fragments in flow-cell channels along with DNA polymerase. Bridge amplification extends cluster strands with all four fluorescently labeled nucleotides for sequencing.
3) Sequencing-by-ligation approaches, *e.g.* implemented in the SOLid^{™} platform of Applied Biosystems (now Life Technologies Corporation, Carlsbad, California). In this technology, a pool of all possible oligonucleotides of a fixed length are labeled according to the sequenced position. Oligonucleotides are annealed and ligated; the preferential ligation by DNA ligase for matching sequences results in a signal informative of the nucleotide at that position. Before sequencing, the DNA is amplified by emulsion PCR. The resulting bead, each containing only copies of the same DNA molecule, are deposited on a glass slide. As a second example, he Polonator^{™} G.007 platform of Dover Systems (Salem, New Hampshire) also employs a sequencing-by-ligation approach by using a randomly arrayed, bead-based, emulsion PCR to amplify DNA fragments for parallel sequencing.
4) Single-molecule sequencing technologies such as *e.g.* implemented in the PacBio RS system of Pacific Biosciences (Menlo Park, California) or in the HeliScope^{™} platform of Helicos Biosciences (Cambridge, Massachusetts). The distinct characteristic of this technology is its ability to sequence single DNA or RNA molecules without amplification, defined as Single-Molecule Real Time (SMRT) DNA sequencing. For example, HeliScope uses a highly sensitive fluorescence detection system to directly detect each nucleotide as it is synthesized. A similar approach based on fluorescence resonance energy transfer (FRET) has been developed from Visigen Biotechnology (Houston, Texas). Other fluorescence-based single-molecule techniques are from U.S. Genomics (GeneEngine^{™}) and Genovoxx (AnyGene^{™}).
5) Nano-technologies for single-molecule sequencing in which various nanostructures are used which are *e.g.* arranged on a chip to monitor the movement of a polymerase molecule on a single strand during replication. Non-limiting examples for approaches based on nano-technologies are the GridON^{™} platform of Oxford Nanopore Technologies (Oxford, UK), the hybridization-assisted nano-pore sequencing (HANS^{™}) platforms developed by Nabsys (Providence, Rhode Island), and the proprietary ligase-based DNA sequencing platform with DNA nanoball (DNB) technology called combinatorial probe-anchor ligation (cPAL^{™}).
6) Electron microscopy based technologies for single-molecule sequencing, *e.g.* those developed by LightSpeed Genomics (Sunnyvale, California) and Halcyon Molecular (Redwood City, California)
7) Ion semiconductor sequencing which is based on the detection of hydrogen ions that are released during the polymerisation of DNA. For example, Ion Torrent Systems (San Francisco, California) uses a high-density array of micro-machined wells to perform this biochemical process in a massively parallel way. Each well holds a different DNA template. Beneath the wells is an ion-sensitive layer and beneath that a proprietary Ion sensor.

In one embodiment, whether a disease-specific mutation occurred can be determined by a method relating to determining that a site in the normal genome is consistent with a homozygous genotype as reflected by a normal allele and three noise alleles, and an ideal noise distribution and declaring a mutation where the corresponding site in the tumor genome is inconsistent with the homozygous genotype and an ideal noise distribution, wherein reads are consistent with an ideal noise distribution if the reads map to each of the noise alleles with a probability of one third of an error rate per base, as disclosed in the International PCT Patent Application entitled "Highly Accurate Mutation Detection, In Particular for Personalized Therapeutics" filed on even date herewith, the disclosure of which is incorporated by reference herein in its entirety.

Preferably, DNA and RNA preparations serve as starting material for NGS. Such nucleic acids can be easily obtained from samples such as biological material, *e.g.* from fresh, flash-frozen or formalin-fixed paraffin embedded tumor tissues (FFPE) or from freshly isolated cells or from CTCs which are present in the peripheral blood of patients. Normal non-mutated genomic DNA or RNA can be extracted from normal, somatic tissue, however germline cells are preferred in the context of the present invention. Germline DNA or RNA is extracted from peripheral blood mononuclear cells (PBMCs) in patients with non-hematological malignancies. Although nucleic acids extracted from FFPE tissues or freshly isolated single cells are highly fragmented, they are suitable for NGS applications.

Several targeted NGS methods for exome sequencing are described in the literature (for review see, *e.g.,* Teer and Mullikin, 2010, Human Mol Genet 19(2):R145-51), all of which can be used in conjunction with the present invention. Many of these methods (described *e.g.* as genome capture, genome partitioning, genome enrichment etc.) use hybridization techniques and include array-based (*e.g.,* Hodges et al., 2007, Nat. Genet. 39:1522-1527) and liquid-based (*e.g.,* Choi et al., 2009, Proc. Natl. Acad. Sci USA 106:19096-19101) hybridization approaches. Commercial kits for DNA sample preparation and subsequent exome capture are also available: for example, Illumina Inc. (San Diego, California) offers the TruSeq^{™} DNA Sample Preparation Kit and the Exome Enrichment Kit TruSeq^{™} Exome Enrichment Kit.

In order to reduce the number of false positive findings in detecting cancer specific somatic mutations or sequence differences when comparing *e.g.* the sequence of a tumor sample to the sequence of a reference sample such as the sequence of a germ line sample it is preferred to determine the sequence in replicates of one or both of these sample types. Thus, it is preferred that the sequence of a reference sample such as the sequence of a germ line sample is determined twice, three times or more. Alternatively or additionally, the sequence of a tumor sample is determined twice, three times or more. It may also be possible to determine the sequence of a reference sample such as the sequence of a germ line sample and/or the sequence of a tumor sample more than once by determining at least once the sequence in genomic DNA and determining at least once the sequence in RNA of said reference sample and/or of said tumor sample. For example, by determining the variations between replicates of a reference sample such as a germ line sample the expected rate of false positive (FDR) somatic mutations as a statistical quantity can be estimated. Technical repeats of a sample should generate identical results and any detected mutation in this "same vs. same comparison" is a false positive. In particular, to determine the false discovery rate for somatic mutation detection in a tumor sample relative to a reference sample, a technical repeat of the reference sample can be used as a reference to estimate the number of false positives. Furthermore, various quality related metrics (*e.g.* coverage or SNP quality) may be combined into a single quality score using a machine learning approach. Optionally, for a given somatic variation all other variations with an exceeding quality score may be counted, which enables a ranking of all variations in a dataset.

In the context of the present invention, the term "RNA" relates to a molecule which comprises at least one ribonucleotide residue and preferably being entirely or substantially composed of ribonucleotide residues. "Ribonucleotide" relates to a nucleotide with a hydroxyl group at the 2'-position of a β-D-ribofuranosyl group. The term "RNA" comprises double-stranded RNA, single-stranded RNA, isolated RNA such as partially or completely purified RNA, essentially pure RNA, synthetic RNA, and recombinantly generated RNA such as modified RNA which differs from naturally occurring RNA by addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

The term "RNA" includes and preferably relates to "mRNA". The term "mRNA" means "messenger-RNA" and relates to a "transcript" which is generated by using a DNA template and encodes a peptide or polypeptide. Typically, an mRNA comprises a 5'-UTR, a protein coding region, and a 3'-UTR. mRNA only possesses limited half-life in cells and *in vitro.* In the context of the present invention, mRNA may be generated by *in vitro* transcription from a DNA template. The *in vitro* transcription methodology is known to the skilled person. For example, there is a variety of *in vitro* transcription kits commercially available.

The stability and translation efficiency of RNA may be modified as required. For example, RNA may be stabilized and its translation increased by one or more modifications having a stabilizing effects and/or increasing translation efficiency of RNA. Such modifications are described, for example, in PCT/EP2006/009448 incorporated herein by reference. In order to increase expression of the RNA used in embodiments of the present invention, it may be modified within the coding region, *i.e.* the sequence encoding the expressed peptide or protein, preferably without altering the sequence of the expressed peptide or protein, so as to increase the GC-content to increase mRNA stability and to perform a codon optimization and, thus, enhance translation in cells.

The term "modification" in the context of the RNA used in the present invention includes any modification of an RNA which is not naturally present in said RNA.

In one embodiment of the invention, the RNA used according to the invention does not have uncapped 5'-triphosphates. Removal of such uncapped 5'-triphosphates can be achieved by treating RNA with a phosphatase.

The RNA according to the invention may have modified ribonucleotides in order to increase its stability and/or decrease cytotoxicity. For example, in one embodiment, in the RNA used according to the invention 5-methylcytidine is substituted partially or completely, preferably completely, for cytidine. Alternatively or additionally, in one embodiment, in the RNA used according to the invention pseudouridine is substituted partially or completely, preferably completely, for uridine.

In one embodiment, the term "modification" relates to providing an RNA with a 5'-cap or 5'-cap analog. The term "5'-cap" refers to a cap structure found on the 5'-end of an mRNA molecule and generally consists of a guanosine nucleotide connected to the mRNA via an unusual 5' to 5' triphosphate linkage. In one embodiment, this guanosine is methylated at the 7-position. The term "conventional 5'-cap" refers to a naturally occurring RNA 5'-cap, preferably to the 7-methylguanosine cap (m⁷G). In the context of the present invention, the term "5'-cap" includes a 5'-cap analog that resembles the RNA cap structure and is modified to possess the ability to stabilize RNA and/or enhance translation of RNA if attached thereto, preferably *in vivo* and/or in a cell.

Providing an RNA with a 5'-cap or 5'-cap analog may be achieved by *in vitro* transcription of a DNA template in presence of said 5'-cap or 5'-cap analog, wherein said 5'-cap is co-transcriptionally incorporated into the generated RNA strand, or the RNA may be generated, for example, by *in vitro* transcription, and the 5'-cap may be attached to the RNA post-transcriptionally using capping enzymes, for example, capping enzymes of vaccinia virus.

The RNA may comprise further modifications. For example, a further modification of the RNA used in the present invention may be an extension or truncation of the naturally occurring poly(A) tail or an alteration of the 5'- or 3'-untranslated regions (UTR) such as introduction of a UTR which is not related to the coding region of said RNA, for example, the exchange of the existing 3'-UTR with or the insertion of one or more, preferably two copies of a 3'-UTR derived from a globin gene, such as alpha2-globin, alpha1-globin, beta-globin, preferably beta-globin, more preferably human beta-globin.

RNA having an unmasked poly-A sequence is translated more efficiently than RNA having a masked poly-A sequence. The term "poly(A) tail" or "poly-A sequence" relates to a sequence of adenyl (A) residues which typically is located on the 3'-end of a RNA molecule and "unmasked poly-A sequence" means that the poly-A sequence at the 3' end of an RNA molecule ends with an A of the poly-A sequence and is not followed by nucleotides other than A located at the 3' end, *i.e.* downstream, of the poly-A sequence. Furthermore, a long poly-A sequence of about 120 base pairs results in an optimal transcript stability and translation efficiency of RNA.

Therefore, in order to increase stability and/or expression of the RNA used according to the present invention, it may be modified so as to be present in conjunction with a poly-A sequence, preferably having a length of 10 to 500, more preferably 30 to 300, even more preferably 65 to 200 and especially 100 to 150 adenosine residues. In an especially preferred embodiment the poly-A sequence has a length of approximately 120 adenosine residues. To further increase stability and/or expression of the RNA used according to the invention, the poly-A sequence can be unmasked.

In addition, incorporation of a 3'-non translated region (UTR) into the 3'-non translated region of an RNA molecule can result in an enhancement in translation efficiency. A synergistic effect may be achieved by incorporating two or more of such 3'-non translated regions. The 3'-non translated regions may be autologous or heterologous to the RNA into which they are introduced. In one particular embodiment the 3'-non translated region is derived from the human β-globin gene.

A combination of the above described modifications, *i.e.* incorporation of a poly-A sequence, unmasking of a poly-A sequence and incorporation of one or more 3'-non translated regions, has a synergistic influence on the stability of RNA and increase in translation efficiency.

The term "stability" of RNA relates to the "half-life" of RNA. "Half-life" relates to the period of time which is needed to eliminate half of the activity, amount, or number of molecules. In the context of the present invention, the half-life of an RNA is indicative for the stability of said RNA. The half-life of RNA may influence the "duration of expression" of the RNA. It can be expected that RNA having a long half-life will be expressed for an extended time period.

Of course, if it is desired to decrease stability and/or translation efficiency of RNA, it is possible to modify RNA so as to interfere with the function of elements as described above increasing the stability and/or translation efficiency of RNA.

The term "expression" is used in its most general meaning and comprises the production of RNA and/or peptides or polypeptides, e.g. by transcription and/or translation. With respect to RNA, the term "expression" or "translation" relates in particular to the production of peptides or polypeptides. It also comprises partial expression of nucleic acids. Moreover, expression can be transient or stable.

The term expression also includes an "aberrant expression" or "abnormal expression". "Aberrant expression" or "abnormal expression" means that expression is altered, preferably increased, compared to a reference, *e.g.* a state in a subject not having a disease associated with aberrant or abnormal expression of a certain protein, *e.g.,* a tumor antigen. An increase in expression refers to an increase by at least 10%, in particular at least 20%, at least 50% or at least 100%, or more. In one embodiment, expression is only found in a diseased tissue, while expression in a healthy tissue is repressed.

The term "specifically expressed" means that a protein is essentially only expressed in a specific tissue or organ. For example, a tumor antigen specifically expressed in gastric mucosa means that said protein is primarily expressed in gastric mucosa and is not expressed in other tissues or is not expressed to a significant extent in other tissue or organ types. Thus, a protein that is exclusively expressed in cells of the gastric mucosa and to a significantly lesser extent in any other tissue, such as testis, is specifically expressed in cells of the gastric mucosa. In some embodiments, a tumor antigen may also be specifically expressed under normal conditions in more than one tissue type or organ, such as in 2 or 3 tissue types or organs, but preferably in not more than 3 different tissue or organ types. In this case, the tumor antigen is then specifically expressed in these organs. For example, if a tumor antigen is expressed under normal conditions preferably to an approximately equal extent in lung and stomach, said tumor antigen is specifically expressed in lung and stomach.

In the context of the present invention, the term "transcription" relates to a process, wherein the genetic code in a DNA sequence is transcribed into RNA. Subsequently, the RNA may be translated into protein. According to the present invention, the term "transcription" comprises *"in vitro* transcription", wherein the term *"in vitro* transcription" relates to a process wherein RNA, in particular mRNA, is *in vitro* synthesized in a cell-free system, preferably using appropriate cell extracts. Preferably, cloning vectors are applied for the generation of transcripts. These cloning vectors are generally designated as transcription vectors and are encompassed by the term "vector". The RNA used in the present invention preferably is *in vitro* transcribed RNA (IVT-RNA) and may be obtained by *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription is controlled by a T7 or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

The term "translation" relates to the process in the ribosomes of a cell by which a strand of messenger RNA directs the assembly of a sequence of amino acids to make a peptide or polypeptide.

Expression control sequences or regulatory sequences, which in the context of the present invention may be linked functionally with a nucleic acid, can be homologous or heterologous with respect to the nucleic acid. A coding sequence and a regulatory sequence are linked together "functionally" if they are bound together covalently, so that the transcription or translation of the coding sequence is under the control or under the influence of the regulatory sequence. If the coding sequence is to be translated into a functional protein, with functional linkage of a regulatory sequence with the coding sequence, induction of the regulatory sequence leads to a transcription of the coding sequence, without causing a reading frame shift in the coding sequence or inability of the coding sequence to be translated into the desired protein or peptide.

The term "expression control sequence" or "regulatory sequence" comprises, in the context of the invention, promoters, ribosome-binding sequences and other control elements, which control the transcription of a nucleic acid or the translation of the derived RNA. In certain embodiments, the regulatory sequences can be controlled. The precise structure of regulatory sequences can vary depending on the species or depending on the cell type, but generally comprises 5'-untranscribed and 5'- and 3'-untranslated sequences, which are involved in the initiation of transcription or translation, such as TATA-box, capping-sequence, CAAT-sequence and the like. In particular, 5'-untranscribed regulatory sequences comprise a promoter region that includes a promoter sequence for transcriptional control of the functionally bound gene. Regulatory sequences can also comprise enhancer sequences or upstream activator sequences.

Preferably, the RNA to be expressed in a cell is introduced into said cell. In one embodiment of the methods according to the invention, the RNA that is to be introduced into a cell is obtained by *in vitro* transcription of an appropriate DNA template.

Terms such as "RNA capable of expressing" and "RNA encoding" are used interchangeably herein and with respect to a particular peptide or polypeptide mean that the RNA, if present in the appropriate environment, preferably within a cell, can be expressed to produce said peptide or polypeptide. Preferably, RNA is able to interact with the cellular translation machinery to provide the peptide or polypeptide it is capable of expressing.

Terms such as "transferring", "introducing" or "transfecting" are used interchangeably herein and relate to the introduction of nucleic acids, in particular exogenous or heterologous nucleic acids, in particular RNA into a cell. According to the present invention, the cell can form part of an organ, a tissue and/or an organism. According to the present invention, the administration of a nucleic acid is either achieved as naked nucleic acid or in combination with an administration reagent. Preferably, administration of nucleic acids is in the form of naked nucleic acids. Preferably, the RNA is administered in combination with stabilizing substances such as RNase inhibitors. The present invention also envisions the repeated introduction of nucleic acids into cells to allow sustained expression for extended time periods.

Cells can be transfected with any carriers with which RNA can be associated, *e.g*. by forming complexes with the RNA or forming vesicles in which the RNA is enclosed or encapsulated, resulting in increased stability of the RNA compared to naked RNA. Useful carriers include, for example, lipid-containing carriers such as cationic lipids, liposomes, in particular cationic liposomes, and micelles, and nanoparticles. Cationic lipids may form complexes with negatively charged nucleic acids. Any cationic lipid may be used.

Preferably, the introduction of RNA which encodes a peptide or polypeptide into a cell, in particular into a cell present *in vivo,* results in expression of said peptide or polypeptide in the cell. In particular embodiments, the targeting of the nucleic acids to particular cells is preferred. In such embodiments, a carrier which is applied for the administration of the nucleic acid to a cell (for example, a retrovirus or a liposome), exhibits a targeting molecule. For example, a molecule such as an antibody which is specific for a surface membrane protein on the target cell or a ligand for a receptor on the target cell may be incorporated into the nucleic acid carrier or may be bound thereto. In case the nucleic acid is administered by liposomes, proteins which bind to a surface membrane protein which is associated with endocytosis may be incorporated into the liposome formulation in order to enable targeting and/or uptake. Such proteins encompass capsid proteins of fragments thereof which are specific for a particular cell type, antibodies against proteins which are internalized, proteins which target an intracellular location, *etc.*

The term "cell" or "host cell" preferably is an intact cell, *i.e.* a cell with an intact membrane that has not released its normal intracellular components such as enzymes, organelles, or genetic material. An intact cell preferably is a viable cell, *i.e.* a living cell capable of carrying out its normal metabolic functions. Preferably said term relates to any cell which can be transformed or transfected with an exogenous nucleic acid. The term "cell" includes prokaryotic cells *(e.g.,* E. coli) or eukaryotic cells *(e.g.,* dendritic cells, B cells, CHO cells, COS cells, K562 cells, HEK293 cells, HELA cells, yeast cells, and insect cells). The exogenous nucleic acid may be found inside the cell (i) freely dispersed as such, (ii) incorporated in a recombinant vector, or (iii) integrated into the host cell genome or mitochondrial DNA. Mammalian cells are particularly preferred, such as cells from humans, mice, hamsters, pigs, goats, and primates. The cells may be derived from a large number of tissue types and include primary cells and cell lines. Specific examples include keratinocytes, peripheral blood leukocytes, bone marrow stem cells, and embryonic stem cells. In further embodiments, the cell is an antigen-presenting cell, in particular a dendritic cell, a monocyte, or macrophage.

A cell which comprises a nucleic acid molecule preferably expresses the peptide or polypeptide encoded by the nucleic acid.

The term "clonal expansion" refers to a process wherein a specific entity is multiplied. In the context of the present invention, the term is preferably used in the context of an immunological response in which lymphocytes are stimulated by an antigen, proliferate, and the specific lymphocyte recognizing said antigen is amplified. Preferably, clonal expansion leads to differentiation of the lymphocytes.

Terms such as "reducing" or "inhibiting" relate to the ability to cause an overall decrease, preferably of 5% or greater, 10% or greater, 20% or greater, more preferably of 50% or greater, and most preferably of 75% or greater, in the level. The term "inhibit" or similar phrases includes a complete or essentially complete inhibition, *i.e.* a reduction to zero or essentially to zero.

Terms such as "increasing", "enhancing", "promoting" or "prolonging" preferably relate to an increase, enhancement, promotion or prolongation by about at least 10%, preferably at least 20%, preferably at least 30%, preferably at least 40%, preferably at least 50%, preferably at least 80%, preferably at least 100%, preferably at least 200% and in particular at least 300%. These terms may also relate to an increase, enhancement, promotion or prolongation from zero or a non-measurable or non-detectable level to a level of more than zero or a level which is measurable or detectable.

According to the present invention, the term "peptide" refers to substances comprising two or more, preferably 3 or more, preferably 4 or more, preferably 6 or more, preferably 8 or more, preferably 10 or more, preferably 13 or more, preferably 16 more, preferably 21 or more and up to preferably 8, 10, 20, 30, 40 or 50, in particular 100 amino acids joined covalently by peptide bonds. The term "polypeptide" or "protein" refers to large peptides, preferably to peptides with more than 100 amino acid residues, but in general the terms "peptide", "polypeptide" and "protein" are synonyms and are used interchangeably herein. According to the invention, the term "modification" or "sequence change" with respect to peptides, polypeptides or proteins relates to a sequence change in a peptide, polypeptide or protein compared to a parental sequence such as the sequence of a wildtype peptide, polypeptide or protein. The term includes amino acid insertion variants, amino acid addition variants, amino acid deletion variants and amino acid substitution variants, preferably amino acid substitution variants. All these sequence changes according to the invention may potentially create new epitopes.

Amino acid insertion variants comprise insertions of single or two or more amino acids in a particular amino acid sequence.

Amino acid addition variants comprise amino- and/or carboxy-terminal fusions of one or more amino acids, such as 1, 2, 3, 4 or 5, or more amino acids.

Amino acid deletion variants are characterized by the removal of one or more amino acids from the sequence, such as by removal of 1, 2, 3, 4 or 5, or more amino acids.

Amino acid substitution variants are characterized by at least one residue in the sequence being removed and another residue being inserted in its place.

According to the invention, a modification or modified peptide used for testing in the methods of the invention may be derived from a protein comprising a modification.

The term "derived" means according to the invention that a particular entity, in particular a particular peptide sequence, is present in the object from which it is derived. In the case of amino acid sequences, especially particular sequence regions, "derived" in particular means that the relevant amino acid sequence is derived from an amino acid sequence in which it is present.

The agents, compositions and methods described herein can be used to treat a subject with a disease, *e.g.,* a disease characterized by the presence of diseased cells expressing an antigen and presenting an antigen peptide. Particularly preferred diseases are cancer diseases. The agents, compositions and methods described herein may also be used for immunization or vaccination to prevent a disease described herein.

One such agent is a vaccine such as a cancer vaccine designed on the basis of suitable neoepitopes that resist immune escape identified by the methods of the present invention.

According to the invention, the term "vaccine" relates to a pharmaceutical preparation (pharmaceutical composition) or product that upon administration induces an immune response, in particular a cellular immune response, which recognizes and attacks a pathogen or a diseased cell such as a cancer cell. A vaccine may be used for the prevention or treatment of a disease. The term "personalized cancer vaccine" or "individualized cancer vaccine" concerns a particular cancer patient and means that a cancer vaccine is adapted to the needs or special circumstances of an individual cancer patient.

The cancer vaccines provided according to the invention when administered to a patent provide one or more T cell epitopes for stimulating, priming and/or expanding T cells specific for the patient's tumor. The T cells are preferably directed against cells expressing antigens from which the T cell epitopes are derived. Thus, the vaccines described herein are preferably capable of inducing or promoting a cellular response, preferably cytotoxic T cell activity, against a cancer disease characterized by presentation of one or more tumor-associated neoantigens with class I MHC. Since a vaccine provided herein will target cancer specific mutations it will be specific for the patient's tumor.

In the context of the present invention, a vaccine relates to a vaccine which when administered to a patient preferably provides one or more T cell epitopes (neoepitopes, suitable neoepitopes, combination of suitable neoepitopes identified herein), such as 2 or more, 5 or more, 10 or more, 15 or more, 20 or more, 25 or more, 30 or more and preferably up to 60, up to 55, up to 50, up to 45, up to 40, up to 35 or up to 30 T cell epitopes, incorporating amino acid modifications or modified peptides predicted as being suitable epitopes. Presentation of these epitopes by cells of a patient, in particular antigen presenting cells, preferably results in T cells targeting the epitopes when bound to MHC and thus, the patient's tumor, preferably the primary tumor as well as tumor metastases, expressing antigens from which the T cell epitopes are derived and presenting the same epitopes on the surface of the tumor cells.

The methods of the invention may comprise the further step of determining the usability of the identified amino acid modifications or modified peptides containing a suitable neoepitope identified herein for cancer vaccination. Thus further steps can involve one or more of the following: (i) assessing whether the modifications are located in known or predicted MHC presented epitopes, (ii) *in vitro* and/or *in silico* testing whether the modifications are located in MHC presented epitopes, *e.g.* testing whether the modifications are part of peptide sequences which are processed into and/or presented as MHC presented epitopes, and (iii) *in vitro* testing whether the envisaged modified epitopes, in particular when present in their natural sequence context, *e.g.* when flanked by amino acid sequences also flanking said epitopes in the naturally occurring protein, and when expressed in antigen presenting cells are able to stimulate T cells such as T cells of the patient having the desired specificity. Such flanking sequences each may comprise 3 or more, 5 or more, 10 or more, 15 or more, 20 or more and preferably up to 50, up to 45, up to 40, up to 35 or up to 30 amino acids and may flank the epitope sequence N-terminally and/or C-terminally.

Modified peptides determined according to the invention may be ranked for their usability as epitopes for cancer vaccination. Thus, in one aspect, the method of the invention comprises a manual or computer-based analytical process in which the identified modified peptides are analyzed and selected for their usability in the respective vaccine to be provided. In a preferred embodiment, said analytical process is a computational algorithm-based process. Preferably, said analytical process comprises determining and/or ranking epitopes according to a prediction of their capacity of being immunogenic.

The epitopes identified according to the invention and provided in a vaccine are preferably present in the form of a polypeptide comprising said epitopes (neoepitopes, suitable neoepitopes, neoepitopes found in a combination of suitable neoepitopes identified herein) such as a polyepitopic polypeptide or a nucleic acid, in particular RNA, encoding said polypeptide. Furthermore, the epitopes may be present in the polypeptide in the form of a vaccine sequence, *i.e.* present in their natural sequence context, *e.g.* flanked by amino acid sequences also flanking said epitopes in the naturally occurring protein. Such flanking sequences each may comprise 5 or more, 10 or more, 15 or more, 20 or more and preferably up to 50, up to 45, up to 40, up to 35 or up to 30 amino acids and may flank the epitope sequence N-terminally and/or C-terminally. Thus, a vaccine sequence may comprise 20 or more, 25 or more, 30 or more, 35 or more, 40 or more and preferably up to 50, up to 45, up to 40, up to 35 or up to 30 amino acids. In one embodiment, the epitopes and/or vaccine sequences are lined up in the polypeptide head-to-tail.

In one embodiment, the epitopes/suitable neoepitopes identified herein and/or vaccine sequences are spaced by linkers, in particular neutral linkers. The term "linker" used in the context of the present invention relates to a peptide added between two peptide domains such as epitopes or vaccine sequences to connect said peptide domains. There is no particular limitation regarding the linker sequence. However, it is preferred that the linker sequence reduces steric hindrance between the two peptide domains, is well translated, and supports or allows processing of the epitopes. Furthermore, the linker should have no or only little immunogenic sequence elements. Linkers preferably should not create non-endogenous epitopes like those generated from the junction suture between adjacent epitopes, which might generate unwanted immune reactions. Therefore, the polyepitopic vaccine should preferably contain linker sequences which are able to reduce the number of unwanted MHC binding junction epitopes. Hoyt et al. (EMBO J. 25(8), 1720-9, 2006) and Zhang et al. (J. Biol. Chem., 279(10), 8635-41, 2004) have shown that glycine-rich sequences impair proteasomal processing and thus the use of glycine rich linker sequences act to minimize the number of linker-contained peptides that can be processed by the proteasome. Furthermore, glycine was observed to inhibit a strong binding in MHC binding groove positions (Abastado et al., 1993, J. Immunol. 151(7):3569-75). Schlessinger et al., 2005, Proteins 61(1):115-26 had found that amino acids glycine and serine included in an amino acid sequence result in a more flexible protein that is more efficiently translated and processed by the proteasome, enabling better access to the encoded epitopes. The linker each may comprise 3 or more, 6 or more, 9 or more, 10 or more, 15 or more, 20 or more and preferably up to 50, up to 45, up to 40, up to 35 or up to 30 amino acids. Preferably the linker is enriched in glycine and/or serine amino acids. Preferably, at least 50%, at least 60%, at least 70%, at least 80%, at least 90%, or at least 95% of the amino acids of the linker are glycine and/or serine. In one preferred embodiment, a linker is substantially composed of the amino acids glycine and serine. In one embodiment, the linker comprises the amino acid sequence (GGS)a(GSS)b(GGG)c(SSG)d(GSG)e wherein a, b, c, d and e is independently a number selected from 0, 1, 2, 3, 4, 5, 6, 7, 8, 9, 10, 11, 12, 13, 14, 15, 16, 17, 18, 19, or 20 and wherein a + b + c + d + e are different from 0 and preferably are 2 or more, 3 or more, 4 or more or 5 or more. In one embodiment, the linker comprises a sequence as described herein including the linker sequences described in the examples such as the sequence GGSGGGGSG.

In one particularly preferred embodiment, a polypeptide incorporating one or more suitable neoepitopes identified by the methods herein, such as a polyepitopic polypeptide, is administered to a patient in the form of a nucleic acid, preferably RNA such as *in vitro* transcribed or synthetic RNA, which may be expressed in cells of a patient such as antigen presenting cells to produce the polypeptide. The present invention also envisions the administration of one or more multiepitopic polypeptides which for the purpose of the present invention are comprised by the term "polyepitopic polypeptide", preferably in the form of a nucleic acid, preferably RNA such as *in vitro* transcribed or synthetic RNA, which may be expressed in cells of a patient such as antigen presenting cells to produce the one or more polypeptides. In the case of an administration of more than one multiepitopic polypeptide the suitable neoepitopes provided by the different multiepitopic polypeptides may be different or partially overlapping. Once present in cells of a patient such as antigen presenting cells the polypeptide according to the invention is processed to produce the suitable neoepitopes identified according to the invention. Administration of a vaccine provided according to the invention may provide MHC class II-presented epitopes that are capable of eliciting a CD4+ helper T cell response against cells expressing antigens from which the MHC presented epitopes are derived. Alternatively or additionally, administration of a vaccine provided according to the invention may provide MHC class I-presented neoepitopes that are capable of eliciting a CD8+ T cell response against cells expressing antigens from which the MHC presented neoepitopes are derived. Furthermore, administration of a vaccine provided according to the invention may provide one or more neoepitopes (including known neoepitopes and suitable neoepitopes identified according to the invention) as well as one or more epitopes not containing cancer specific somatic mutations but being expressed by cancer cells and preferably inducing an immune response against cancer cells, preferably a cancer specific immune response. In one embodiment, administration of a vaccine provided according to the invention provides neoepitopes that are MHC class II-presented epitopes and/or are capable of eliciting a CD4+ helper T cell response against cells expressing antigens from which the MHC presented epitopes are derived as well as epitopes not containing cancer-specific somatic mutations that are MHC class I-presented epitopes and/or are capable of eliciting a CD8+ T cell response against cells expressing antigens from which the MHC presented epitopes are derived. In one embodiment, the epitopes not containing cancer-specific somatic mutations are derived from a tumor antigen. In one embodiment, the neoepitopes and epitopes not containing cancer-specific somatic mutations have a synergistic effect in the treatment of cancer. Preferably, a vaccine provided according to the invention is useful for polyepitopic stimulation of cytotoxic and/or helper T cell responses.

The vaccine provided according to the invention may be a recombinant vaccine.

Another type of agent is an immune cell, such as a T cell expressing a T cell receptor, or a T cell recombinantly expressing a T cell receptor, or expressing an artificial or chimeric T cell receptor (CAR), which receptor is targeted to an antigen, e.g., a suitable neoepitope identified by the methods of the present invention as a suitable disease-specific target, preferably where such neoepitope is expressed on the surface of a cell in a complex with MHC molecules. Preferably, once the immune cell recognizes the antigen by receptor-antigen binding, the immune cell (immunoreactive cell) is stimulated, primed and/or expanded or exerts effector functions of immunoreactive cells as described above.

The term "antigen-specific T cell" or similar terms relate to a T cell which recognizes an antigen, *e.g.,* a suitable neoepitope complexed within MHC class I molecules, and upon binding to said antigen preferably exerts effector functions of T cells as described above. T cells and other lymphoid cells are considered to be specific for antigen if the cells kill target cells expressing the antigen. T cell specificity may be evaluated using any of a variety of standard techniques, for example, within a chromium release assay or proliferation assay. Alternatively, synthesis of lymphokines (such as interferon-y) can be measured.

T cell receptors and other antigen receptors are described *supra.* The term "CAR" (or "chimeric antigen receptor") relates to an artificial receptor comprising a single molecule or a complex of molecules which recognizes, *i.e.,* binds to, a target structure *(e.g.* an antigen) on a target cell such as a cancer cell *(e.g.,* by binding of an antigen binding domain to an antigen expressed on the surface of the target cell) and may confer specificity onto an immune effector cell such as a T cell expressing said CAR on the cell surface. Preferably, recognition of the target structure by a CAR results in activation of an immune effector cell expressing said CAR. A CAR may comprise one or more protein units said protein units comprising one or more domains as described herein. The term "CAR" does not include T cell receptors.

In one embodiment, a single-chain variable fragment (scFv) derived from a monoclonal antibody is fused to CD3-zeta transmembrane and endodomain. Such molecules result in the transmission of a zeta signal in response to recognition by the scFv of its antigen target on a target cell and killing of the target cell that expresses the target antigen. Antigen recognition domains which also may be used include among others T cell receptor (TCR) alpha and beta single chains. In fact almost anything that binds a given target with high affinity can be used as an antigen recognition domain.

Following antigen recognition, receptors cluster and a signal is transmitted to the cell. In this respect, a "T cell signaling domain" is a domain, preferably an endodomain, which transmits an activation signal to the T cell after antigen is bound. The most commonly used endodomain component is CD3-zeta.

Adoptive cell transfer therapy with CAR-engineered T cells expressing chimeric antigen receptors is a promising mode of therapy since CAR-modified T cells can be engineered to target virtually any antigen expressed on diseased cells, for example tumor antigens. Preferably, the tumor antigen is a neoepitope resulting from a tumor-specific mutation identified by the methods of the present invention as a suitable tumor-specific target. For example, patient's T cells may be genetically engineered (genetically modified) to express a CAR specifically directed towards a tumor-specific neoepitope complexed with MHC molecules on the surface of the patient's tumor cells, then infused back into the patient.

A CAR may replace the function of a T cell receptor and, in particular, may confer reactivity such as cytolytic activity to a cell such as a T cell. However, in contrast to the binding of the T cell receptor to an antigen peptide-MHC complex as described above, a CAR also may bind to an antigen, in particular when expressed on the cell surface.

According to the invention, CARs may generally comprise three domains. The first domain is the binding domain which recognizes and binds antigen. The second domain is the co-stimulation domain. The co-stimulation domain serves to enhance the proliferation and survival of the cytotoxic lymphocytes upon binding of the CAR to a targeted moiety. The identity of the co-stimulation domain is limited only in that it has the ability to enhance cellular proliferation and survival upon binding of the targeted moiety by the CAR. Suitable co-stimulation domains include CD28, CD137 (4-1BB), a member of the tumor necrosis factor (TNF) receptor family, CD 134 (OX40), a member of the TNFR-superfamily of receptors, and CD278 (ICOS), a CD28-superfamily co-stimulatory molecule expressed on activated T cells. The third domain is the activation signaling domain (or T cell signaling domain). The activation signaling domain serves to activate cytotoxic lymphocytes upon binding of the CAR to antigen. The identity of the activation signaling domain is limited only in that it has the ability to induce activation of the selected cytotoxic lymphocyte upon binding of the antigen by the CAR. Suitable activation signaling domains include the T cell CD3 [zeta] chain and Fc receptor [gamma].

The CARs may comprise the three domains, together in the form of a fusion protein. Such fusion proteins will generally comprise a binding domain, one or more co-stimulation domains, and an activation signaling domain, linked in an N-terminal to C-terminal direction. However, the CARs are not limited to this arrangement and other arrangements are acceptable and include a binding domain, an activation signaling domain, and one or more co-stimulation domains. It will be understood that because the binding domain must be free to bind antigen, the placement of the binding domain in the fusion protein will generally be such that display of the region on the exterior of the cell is achieved. In the same manner, because the co-stimulation and activation signaling domains serve to induce activity and proliferation of the cytotoxic lymphocytes, the fusion protein will generally display these two domains in the interior of the cell. The CARs may include additional elements, such as a signal peptide to ensure proper export of the fusion protein to the cells surface, a transmembrane domain to ensure the fusion protein is maintained as an integral membrane protein, and a hinge domain (or spacer region) that imparts flexibility to the binding domain and allows strong binding to antigen.

The cells used in connection with CARs and other artificial antigen receptors are preferably T cells, in particular cytotoxic lymphocytes, preferably selected from cytotoxic T cells, natural killer (NK) cells, and lymphokine-activated killer (LAK) cells. Upon activation, each of these cytotoxic lymphocytes triggers the destruction of target cells. For example, cytotoxic T cells trigger the destruction of target cells by either or both of the following means. First, upon activation T cells release cytotoxins such as perforin, granzymes, and granulysin. Perforin and granulysin create pores in the target cell, and granzymes enter the cell and trigger a caspase cascade in the cytoplasm that induces apoptosis (programmed cell death) of the cell. Second, apoptosis can be induced via Fas-Fas ligand interaction between the T cells and target cells. The cytotoxic lymphocytes will preferably be autologous cells, although heterologous cells or allogenic cells can be used.

A binding domain for an antigen which may be present within a CAR has the ability to bind to (target) an antigen, *i.e.* the ability to bind to (target) an epitope present in an antigen, preferably the ability to bind to (target) a neoepitope identified by the methods of the present invention as a suitable disease-specific target where the neoepitope is presented in the context of MHC on the surface of the cell. Preferably, a binding domain for an antigen is specific for the antigen.

Another type of agent is an immune cell, such as a lymphoid cell, loaded with a peptide containing a suitable neoepitope identified by the methods of the present invention. In a preferred embodiment, the lymphoid cell is a dendritic cell. In the context of the present invention, lymphoid cells preferably isolated from the patient to be treated are incubated with an antigen to be targeted and the incubated cells are then administered to the patient where an immune response to cells expressing the antigen is induced. Thus, a peptide comprising a suitable epitope can be incubated with dendritic cells and the incubated cells can be administered in order to induce an immune response against cells expressing the suitable neoepitope.

The term "recombinant" in the context of the present invention means "made through genetic engineering". Preferably, a "recombinant entity" such as a recombinant polypeptide in the context of the present invention is not occurring naturally, and preferably is a result of a combination of entities such as amino acid or nucleic acid sequences which are not combined in nature. For example, a recombinant polypeptide in the context of the present invention may contain several amino acid sequences such as neo-epitopes or vaccine sequences derived from different proteins or different portions of the same protein fused together, *e.g*., by peptide bonds or appropriate linkers.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring.

According to the invention, the term "disease" refers to any pathological state, including cancer diseases, in particular those forms of cancer diseases described herein.

The term "normal" refers to the healthy state or the conditions in a healthy subject or tissue, *i.e.,* non-pathological conditions, wherein "healthy" preferably means non-cancerous.

"Disease involving cells expressing an antigen" means that expression of the antigen in cells of a diseased tissue or organ is detected. Expression in cells of a diseased tissue or organ may be increased compared to the state in a healthy tissue or organ. An increase refers to an increase by at least 10%, in particular at least 20%, at least 50%, at least 100%, at least 200%, at least 500%, at least 1000%, at least 10000% or even more. In one embodiment, expression is only found in a diseased tissue, while expression in a healthy tissue is repressed. According to the invention, diseases involving or being associated with cells expressing an antigen include cancer diseases.

Cancer (medical term: malignant neoplasm) is a class of diseases in which a group of cells display uncontrolled growth (division beyond the normal limits), invasion (intrusion on and destruction of adjacent tissues), and sometimes metastasis (spread to other locations in the body via lymph or blood). These three malignant properties of cancers differentiate them from benign tumors, which are self-limited, and do not invade or metastasize. Most cancers form a tumor but some, like leukemia, do not.

Malignant tumor is essentially synonymous with cancer. Malignancy, malignant neoplasm, and malignant tumor are essentially synonymous with cancer.

According to the invention, the term "tumor" or "tumor disease" refers to an abnormal growth of cells (called neoplastic cells, tumorigenous cells or tumor cells) preferably forming a swelling or lesion. By "tumor cell" is meant an abnormal cell that grows by a rapid, uncontrolled cellular proliferation and continues to grow after the stimuli that initiated the new growth cease. Tumors show partial or complete lack of structural organization and functional coordination with the normal tissue, and usually form a distinct mass of tissue, which may be either benign, pre-malignant or malignant.

A benign tumor is a tumor that lacks all three of the malignant properties of a cancer. Thus, by definition, a benign tumor does not grow in an unlimited, aggressive manner, does not invade surrounding tissues, and does not spread to non-adjacent tissues (metastasize).

Neoplasm is an abnormal mass of tissue as a result of neoplasia. Neoplasia (new growth in Greek) is the abnormal proliferation of cells. The growth of the cells exceeds, and is uncoordinated with that of the normal tissues around it. The growth persists in the same excessive manner even after cessation of the stimuli. It usually causes a lump or tumor. Neoplasms may be benign, pre-malignant or malignant.

"Growth of a tumor" or "tumor growth" in the context of the present invention relates to the tendency of a tumor to increase its size and/or to the tendency of tumor cells to proliferate.

For purposes of the present invention, the terms "cancer" and "cancer disease" are used interchangeably with the terms "tumor" and "tumor disease".

Cancers are classified by the type of cell that resembles the tumor and, therefore, the tissue presumed to be the origin of the tumor. These are the histology and the location, respectively.

The term "cancer" according to the invention comprises leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the metastases thereof. Examples thereof are lung carcinomas, mamma carcinomas, prostate carcinomas, colon carcinomas, renal cell carcinomas, cervical carcinomas, or metastases of the cancer types or tumors described above. The term cancer according to the invention also comprises cancer metastases and relapse of cancer.

By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor, *i.e.* a secondary tumor or metastatic tumor, at the target site depends on angiogenesis. Tumor metastasis often occurs even after the removal of the primary tumor because tumor cells or components may remain and develop metastatic potential. In one embodiment, the term "metastasis" according to the invention relates to "distant metastasis" which relates to a metastasis which is remote from the primary tumor and the regional lymph node system.

The cells of a secondary or metastatic tumor are like those in the original tumor. This means, for example, that, if ovarian cancer metastasizes to the liver, the secondary tumor is made up of abnormal ovarian cells, not of abnormal liver cells. The tumor in the liver is then called metastatic ovarian cancer, not liver cancer.

The term "circulating tumor cells" or "CTCs" relates to cells that have detached from a primary tumor or tumor metastases and circulate in the bloodstream. CTCs may constitute seeds for subsequent growth of additional tumors (metastasis) in different tissues. Circulating tumor cells are found in frequencies in the order of 1-10 CTC per mL of whole blood in patients with metastatic disease. Research methods have been developed to isolate CTC. Several research methods have been described in the art to isolate CTCs, e.g. techniques which use of the fact that epithelial cells commonly express the cell adhesion protein EpCAM, which is absent in normal blood cells. Immunomagnetic bead-based capture involves treating blood specimens with antibody to EpCAM that has been conjugated with magnetic particles, followed by separation of tagged cells in a magnetic field. Isolated cells are then stained with antibody to another epithelial marker, cytokeratin, as well as a common leukocyte marker CD45, so as to distinguish rare CTCs from contaminating white blood cells. This robust and semi-automated approach identifies CTCs with an average yield of approximately 1 CTC/mL and a purity of 0.1% (Allard et al., 2004, Clin Cancer Res 10:6897-6904). A second method for isolating CTCs uses a microfluidic-based CTC capture device which involves flowing whole blood through a chamber embedded with 80,000 microposts that have been rendered functional by coating with antibody to EpCAM. CTCs are then stained with secondary antibodies against either cytokeratin or tissue specific markers, such as PSA in prostate cancer or HER2 in breast cancer and are visualized by automated scanning of microposts in multiple planes along three dimensional coordinates. CTC-chips are able to identifying cytokerating-positive circulating tumor cells in patients with a median yield of 50 cells/ml and purity ranging from 1-80% (Nagrath et al., 2007, Nature 450:1235-1239). Another possibility for isolating CTCs is using the CellSearch^{™} Circulating Tumor Cell (CTC) Test from Veridex, LLC (Raritan, NJ) which captures, identifies, and counts CTCs in a tube of blood. The CellSearch^{™} system is a U.S. Food and Drug Administration (FDA) approved methodology for enumeration of CTC in whole blood which is based on a combination of immunomagnetic labeling and automated digital microscopy. There are other methods for isolating CTCs described in the literature all of which can be used in conjunction with the present invention.

A relapse or recurrence occurs when a person is affected again by a condition that affected them in the past. For example, if a patient has suffered from a tumor disease, has received a successful treatment of said disease and again develops said disease said newly developed disease may be considered as relapse or recurrence. However, according to the invention, a relapse or recurrence of a tumor disease may but does not necessarily occur at the site of the original tumor disease. Thus, for example, if a patient has suffered from ovarian tumor and has received a successful treatment a relapse or recurrence may be the occurrence of an ovarian tumor or the occurrence of a tumor at a site different to ovary. A relapse or recurrence of a tumor also includes situations wherein a tumor occurs at a site different to the site of the original tumor as well as at the site of the original tumor. Preferably, the original tumor for which the patient has received a treatment is a primary tumor and the tumor at a site different to the site of the original tumor is a secondary or metastatic tumor.

The term "immune response" relates to a reaction of the immune system such as to immunogenic organisms, such as bacteria or viruses, cells or substances. The term "immune response" includes the innate immune response and the adaptive immune response. Preferably, the immune response is related to an activation of immune cells, an induction of cytokine biosynthesis and/or antibody production.

It is preferred that the immune response induced by the compositions of the present invention comprises the steps of activation of antigen presenting cells, such as dendritic cells and/or macrophages, presentation of an antigen or fragment thereof by said antigen presenting cells and activation of cytotoxic T cells due to this presentation.

The term "immune cells" refers to cells of the immune system involved in defending the body of an individual. The term "immune cells" encompasses specific types of immune cells and their precursors including leucocytes comprising macrophages, monocytes (precursors of macrophages), granulocytes such as neutrophils, eosinophils and basophils, dendritic cells, mast cells, and lymphocytes such as B cells, T cells and natural killer (NK) cells. Macrophages, monocytes (precursors of macrophages), neutrophils, dendritic cells, and mast cells are phagocytic cells.

The term "immunotherapy" relates to the treatment of a disease or condition by inducing, enhancing, or suppressing an immune response. Immunotherapies designed to elicit or amplify an immune response are classified as activation immunotherapies, while immunotherapies that reduce or suppress an immune response are classified as suppression immunotherapies. The term "immunotherapy" includes antigen immunization or antigen vaccination, or tumor immunization or tumor vaccination. The term "immunotherapy" also relates to the manipulation of immune responses such that inappropriate immune responses are modulated into more appropriate ones in the context of autoimmune diseases such as rheumatic arthritis, allergies, diabetes or multiple sclerosis.

The terms "immunization" or "vaccination" describe the process of administering an antigen to an individual with the purpose of inducing an immune response, for example, for therapeutic or prophylactic reasons.

By "treat" is meant to administer a compound or composition as described herein to a subject in order to prevent or eliminate a disease, including reducing the size of a tumor or the number of tumors in a subject; arrest or slow a disease in a subject; inhibit or slow the development of a new disease in a subject; decrease the frequency or severity of symptoms and/or recurrences in a subject who currently has or who previously has had a disease; and/or prolong, *i.e.* increase the lifespan of the subject. In particular, the term "treatment of a disease" includes curing, shortening the duration, ameliorating, preventing, slowing down or inhibiting progression or worsening, or preventing or delaying the onset of a disease or the symptoms thereof.

By "being at risk" is meant a subject, *i.e.* a patient, that is identified as having a higher than normal chance of developing a disease, in particular cancer, compared to the general population. In addition, a subject who has had, or who currently has, a disease, in particular cancer, is a subject who has an increased risk for developing a disease, as such a subject may continue to develop a disease. Subjects who currently have, or who have had, a cancer also have an increased risk for cancer metastases.

A prophylactic administration of an immunotherapy, for example, a prophylactic administration of the composition of the invention, preferably protects the recipient from the development of a disease. A therapeutic administration of an immunotherapy, for example, a therapeutic administration of the composition of the invention, may lead to the inhibition of the progress/growth of the disease. This comprises the deceleration of the progress/growth of the disease, in particular a disruption of the progression of the disease, which preferably leads to elimination of the disease.

Immunotherapy may be performed using any of a variety of techniques, in which agents provided herein function to remove diseased cells from a patient. Such removal may take place as a result of enhancing or inducing an immune response in a patient specific for an antigen or a cell expressing an antigen.

Within certain embodiments, immunotherapy may be active immunotherapy, in which treatment relies on the *in vivo* stimulation of the endogenous host immune system to react against diseased cells with the administration of immune response-modifying agents (such as polypeptides and nucleic acids as provided herein).

The agents and compositions provided herein may be used alone or in combination with conventional therapeutic regimens such as surgery, irradiation, chemotherapy and/or bone marrow transplantation (autologous, syngeneic, allogeneic or unrelated).

The term *"in vivo"* relates to the situation in a subject.

The terms "subject", "individual", "organism" or "patient" relate to vertebrates, particularly mammals. For example, mammals in the context of the present invention are humans, non-human primates, domesticated mammals such as dogs, cats, sheep, cattle, goats, pigs, horses etc., laboratory animals such as mice, rats, rabbits, guinea pigs, etc. as well as animals in captivity such as animals of zoos. The terms also relate to non-mammalian vertebrates such as birds (particularly domesticated birds such as chicken, ducks, geese, turkeys) and to fish (particularly farmed fish, *e.g.* salmon or catfish). The term "animal" as used herein also includes humans.

The term "autologous" is used to describe anything that is derived from the same subject. For example, "autologous transplant" refers to a transplant of tissue or organs derived from the same subject. Such procedures are advantageous because they overcome the immunological barrier which otherwise results in rejection.

The term "heterologous" is used to describe something consisting of multiple different elements. As an example, the transfer of one individual's bone marrow into a different individual constitutes a heterologous transplant. A heterologous gene is a gene derived from a source other than the subject.

As part of the composition for an immunization or a vaccination, preferably one or more agents as described herein are administered together with one or more adjuvants for inducing an immune response or for increasing an immune response. The term "adjuvant" relates to compounds which prolongs or enhances or accelerates an immune response. The composition of the present invention preferably exerts its effect without addition of adjuvants. Still, the composition of the present application may contain any known adjuvant. Adjuvants comprise a heterogeneous group of compounds such as oil emulsions *(e.g.,* Freund's adjuvants), mineral compounds (such as alum), bacterial products (such as *Bordetella pertussis* toxin), liposomes, and immune-stimulating complexes. Examples for adjuvants are monophosphoryl-lipid-A (MPL SmithKline Beecham). Saponins such as QS21 (SmithKline Beecham), DQS21 (SmithKline Beecham; WO 96/33739), QS7, QS17, QS18, and QS-L1 (So et al., 1997, Mol. Cells 7: 178-186), incomplete Freund's adjuvants, complete Freund's adjuvants, vitamin E, montanid, alum, CpG oligonucleotides (Krieg et al., 1995, Nature 374: 546-549), and various water-in-oil emulsions which are prepared from biologically degradable oils such as squalene and/or tocopherol.

Other substances which stimulate an immune response of the patient may also be administered. It is possible, for example, to use cytokines in a vaccination, owing to their regulatory properties on lymphocytes. Such cytokines comprise, for example, interleukin-12 (IL-12) which was shown to increase the protective actions of vaccines (see, Hall, 1995, IL-12 at the crossroads, Science 268:1432-1434), GM-CSF and IL-18.

There are a number of compounds which enhance an immune response and which therefore may be used in a vaccination. Said compounds comprise co-stimulating molecules provided in the form of proteins or nucleic acids such as B7-1 and B7-2 (CD80 and CD86, respectively).

According to the invention, a "tumor specimen" is a sample such as a bodily sample containing tumor or cancer cells such as circulating tumor cells (CTC), in particular a tissue sample, including body fluids, and/or a cellular sample. According to the invention, a "non-tumorous specimen" is a sample such as a bodily sample not containing tumor or cancer cells such as circulating tumor cells (CTC), in particular a tissue sample, including body fluids, and/or a cellular sample. Such bodily samples may be obtained in the conventional manner such as by tissue biopsy, including punch biopsy, and by taking blood, bronchial aspirate, sputum, urine, feces or other body fluids. According to the invention, the term "sample" also includes processed samples such as fractions or isolates of biological samples, *e.g.* nucleic acid or cell isolates.

The therapeutically active agents, vaccines and compositions described herein may be administered via any conventional route, including by injection or infusion. The administration may be carried out, for example, orally, intravenously, intraperitoneally, intramuscularly, subcutaneously or transdermally. In one embodiment, administration is carried out intranodally such as by injection into a lymph node. Other forms of administration envision the *in vitro* transfection of antigen presenting cells such as dendritic cells with nucleic acids described herein followed by administration of the antigen presenting cells.

The agents described herein are administered in effective amounts. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition.

An effective amount of an agent described herein will depend on the condition to be treated, the severity of the disease, the individual parameters of the patient, including age, physiological condition, size and weight, the duration of treatment, the type of an accompanying therapy (if present), the specific route of administration and similar factors. Accordingly, the doses administered of the agents described herein may variously depend on such parameters. In the case that a reaction in a patient is insufficient with an initial dose, higher doses (or effectively higher doses achieved by a different, more localized route of administration) may be used.

The term "pharmaceutically acceptable" refers to the non-toxicity of a material which does not interact with the action of the active component of the pharmaceutical composition.

The pharmaceutical compositions of the present invention may contain salts, buffers, preserving agents, carriers and optionally other therapeutic agents. Preferably, the pharmaceutical compositions of the present invention comprise one or more pharmaceutically acceptable carriers, diluents and/or excipients.

The term "excipient" is intended to indicate all substances in a pharmaceutical composition which are not active ingredients such as binders, lubricants, thickeners, surface active agents, preservatives, emulsifiers, buffers, flavoring agents, or colorants.

The term "diluent" relates a diluting and/or thinning agent. Moreover, the term "diluent" includes any one or more of fluid, liquid or solid suspension and/or mixing media.

The term "carrier" relates to one or more compatible solid or liquid fillers or diluents, which are suitable for an administration to a human. The term "carrier" relates to a natural or synthetic organic or inorganic component which is combined with an active component in order to facilitate the application of the active component. Preferably, carrier components are sterile liquids such as water or oils, including those which are derived from mineral oil, animals, or plants, such as peanut oil, soy bean oil, sesame oil, sunflower oil, etc. Salt solutions and aqueous dextrose and glycerin solutions may also be used as aqueous carrier compounds.

Pharmaceutically acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include, for example, magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Examples of suitable diluents include ethanol, glycerol and water.

Pharmaceutical carriers, excipients or diluents can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

In one embodiment, the composition is an aqueous composition. The aqueous composition may optionally comprise solutes, *e.g*. salts. In one embodiment, the composition is in the form of a freeze-dried composition. A freeze-dried composition is obtainable by freeze-drying a respective aqueous composition.

The agents and compositions provided herein may be used alone or in combination with other therapeutic regimens such as surgery, irradiation, chemotherapy and/or bone marrow transplantation (autologous, syngeneic, allogeneic or unrelated).

The present invention is described in detail and is illustrated by the figures and examples, which are used only for illustration purposes and are not meant to be limiting. Owing to the description and the examples, further embodiments which are likewise included in the invention are accessible to the skilled worker.

### FIGURES

**Figures 1a** **and** **1b** Glioblastoma sample with high focal amplification of the epidermal growth factor receptor gene (EGFR). Fig. 1a: A graphical representation of local genes surrounding EGFR on chromosome 7. Fig. 1b: Listing of the 12 single nucleotide variations with the highest absolute copy numbers.
**Figure 2** A listing of a number of genes having a disease-specific mutation of a melanoma sample sorted by zygosity (Cx).
**Figure 3** A listing of a number of genes of a melanoma sample in which all copies have the disease-specific mutation (fractional zygosity is equal to 1), of which three of the genes are essential genes in humans or inferred to be essential genes in humans.

Abbreviations for the figures: chr_pos chromosomal position; CN absolute copy number; Cx zygosity; EC error correction of the absolute copy number; VAF variant allele frequency; rho estimated percent of tumor cells containing the mutated allele (SNV); FLRT+u confidence score in the mutation; Site classification is the confidence class of the mutation; essential gene, Y if the gene is found to be essential..

### EXAMPLE 1 Targeting disease-specific mutations in genes with high copy number

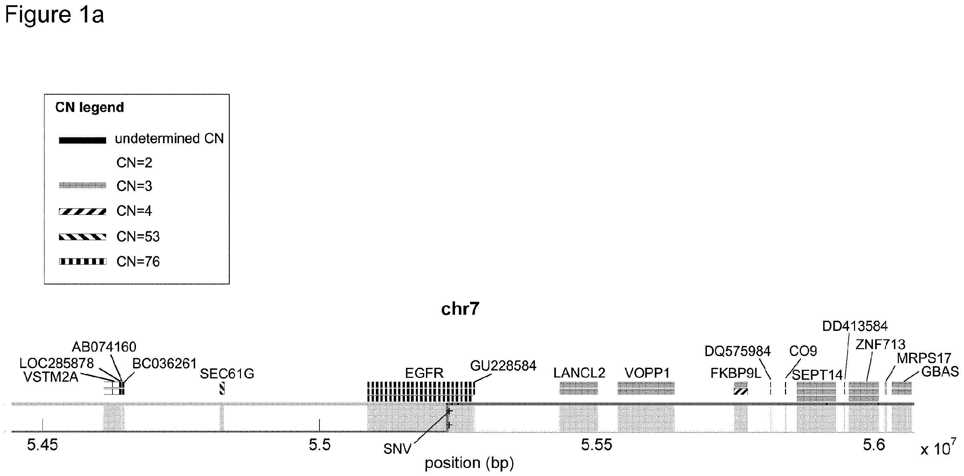

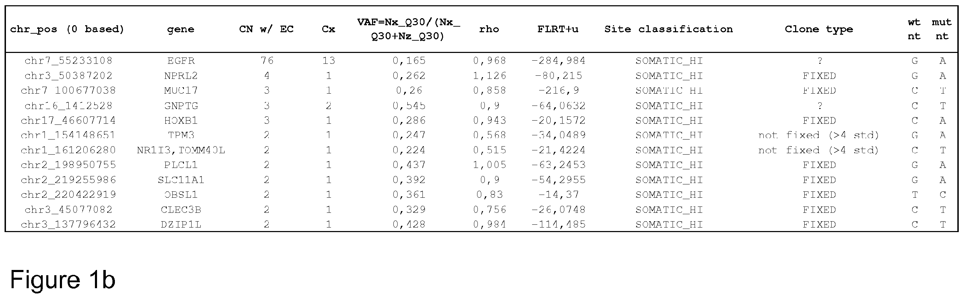

Genomic information for glioblastoma sample (Chin et al., 2008, Comprehensive genomic characterization defines human glioblastoma genes and core pathways, Nature 455:1061-1068) was analyzed by looking for genes having a high copy number and in which at least one copy of the gene contained a disease-specific mutation. The quality analysis indicated that there was a high fidelity of copy number assignment for the 11,574 individual genes analyzed, and the ploidy of the genome of the sample was determined to be 1.95. Figure 1a shows a graphical representation of the local genes surrounding epidermal growth factor receptor (EGFR) on chromosome 7, which is a known driver gene and have been a target for treatment. It was shown that EGFR in this genome had an error corrected absolute copy number of 76, of which 13 copies contained the disease-specific single nucleotide variation. Figure 1b provides a list of the genes in this genomic sample with the highest absolute copy number. There are four additional genes having an absolute copy number greater than 2. Indeed, EGFR amplification is a known genetic hallmark of primary glioblastomas (Benito et al., 2009, Neuropathology 30 (4):392-400) and this gene has been considered as a target for treatment (Taylor, 2012, Curr Cancer Drug Targets. Mar; 12(3):197-209).

### EXAMPLE 2 Targeting disease-specific mutations with a high zygosity

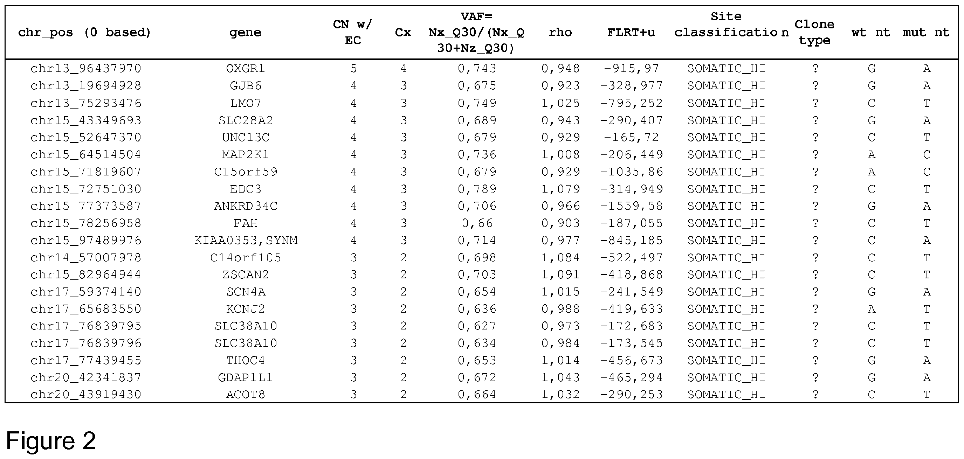

An exome obtained from a sample of melanoma cells from a tumor in a human was analyzed by looking for genes in which at least one copy of the gene has a disease-specific mutation and looking at the number of copies of the gene having the disease-specific mutation as well as the total number of copies of the gene, whether the gene has the mutation or not. Figure 2 provides a list of genes sorted by zygosity in which the disease-specific mutation is found on multiple copies of the gene. For example, the disease-specific mutation in the OXGR1 gene has the highest zygosity (4), and in particular also has the highest fractional zygosity of 4/5 or 0.8 since there are a total of 5 copies of the OXGR1 gene and 4 of which copies contain the disease-specific mutation. The list provides 10 additional genes in which 3 copies of the gene out of a total of 4 copies have the mutation, indicating that the disease-specific mutation in these genes has a fractional zygosity of 3/4 or 0.75. The remaining listed genes have mutations that have a fractional zygosity of 2/3 or 0.66 since 2 copies out of a total of 3 copies have the mutation.

### EXAMPLE 3 Targeting disease-specific mutations present in all copies of an essential gene

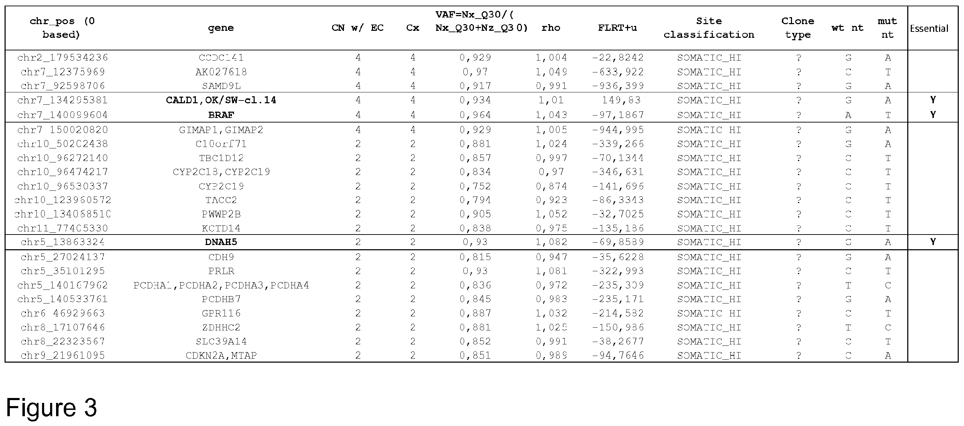

An exome obtained from a sample of melanoma cells from a tumor in a human was analyzed by looking for genes in which all copies of the gene have the same disease-specific mutation and determining which of these genes is an essential gene. The genes were determined to be essential by inferring their essentiality in humans from the knowledge that they are essential in mice (Georgi et al., 2013, From mouse to human: evolutionary genomics analysis of human orthologs of essential genes, PLoS Genetics 9 (5):e1003484; Liao et al., 2007, Mouse duplicate genes are as essential as singletons, Trends Genet. 23:378-381). Figure 3 lists a number of genes in which all copies of the gene have the same disease-specific mutation. Moreover, the three highlighted genes were determined to be essential by inferring their essentiality from mouse data.

The invention provides, in particular, the following:
1. A method for determining the suitability of a neoepitope resulting from a disease-specific mutation at an allele in a gene (mutated allele) as a disease-specific target comprising determining, in a diseased cell or population of diseased cells, the copy number of the mutated allele encoding the neoepitope.
2. The method according to item 1, wherein a high copy number of the mutated allele indicates the suitability of the neoepitope as a disease-specific target.
3. The method according to item 2, wherein the higher the copy number of the mutated allele, the higher the suitability of the neoepitope as a disease-specific target.
4. The method according to item 1, wherein when the copy number of the mutated allele is greater than 2 indicates the suitability of the neoepitope as a disease-specific target.
5. The method according to item 4, wherein when the copy number of the mutated allele is greater than 3, 4, 5, 6, 7, 8, 9, 10, 20, 30, 40, 50, 60, 70, 80, 90, or is greater than 100 indicates the suitability of the neoepitope as a disease-specific target.
6. The method according to any one of items 1 to 5, wherein the mutated allele is found in a high fraction of copies of the gene of which at least one copy has the mutated allele (fractional zygosity), which fractional zygosity is the ratio of the copy number of the mutated allele over the total number of copies of the nucleotide site to which the mutation maps.
7. The method according to item 6, wherein the higher the fractional zygosity, the higher the suitability of the neoepitope as a disease-specific target.
8. The method according to item 6 or 7, wherein the fractional zygosity is greater than 0.5, preferably the fractional zygosity is 1.
9. The method according to any one of items 1 to 8, wherein the copy number of the mutated allele and/or the fractional zygosity and/or the total number of copies of the nucleotide site to which the mutated allele maps is found in a high fraction of diseased cells.
10. The method according to item 9, wherein the higher the fraction of diseased cells having the copy number of the mutated allele and/or the fractional zygosity and/or the total number of copies of the nucleotide site to which the mutated allele maps, the higher the suitability of the neoepitope as a disease-specific target.
11. The method according to item 9 or 10, wherein the fraction of diseased cells is 1.
12. The method according to any one of items 1 to 11, wherein the gene is a driver gene whose expression results in transformation of the cell into a cancerous phenotype or whose lack of expression results in a cancerous cell losing its cancerous phenotype.
13. The method according to any one of items 1 to 11, wherein the gene is an essential gene.
14. The method according to item 13, wherein the essential gene is a gene, which when silenced or its expression is reduced, at least results in impaired growth or reduced fitness of a cell in which the essential gene is expressed, preferably the diseased cell.
15. The method according to item 13, wherein the essential gene is expressed in a wide variety of different tissues and is expressed with a minimal RPKM threshold greater than 0.
16. A method for determining the suitability of a neoepitope resulting from a disease-specific mutation in a gene as a disease-specific target comprising determining, in a diseased cell or population of diseased cells, the copy number of the gene, of which at least one copy has the disease-specific mutation.
17. The method according to item 16, wherein a high copy number of the gene indicates the suitability of the neoepitope as a disease-specific target.
18. The method according to item 16 or 17, wherein the higher the copy number of the gene, the higher the suitability of the neoepitope as a disease-specific target.
19. The method according to any one of items 16 to 18, wherein the gene is a driver gene whose expression results in transformation of the cell into a cancerous phenotype or whose lack of expression results in a cancerous cell losing its cancerous phenotype.
20. The method according to any one of items 16 to 19, wherein the copy number of the gene is found in a high fraction of diseased cells.
21. The method according to item 20, wherein the higher the fraction of diseased cells having the copy number, the higher the suitability of the neoepitope as a disease-specific target.
22. The method according to item 20 or 21, wherein the fraction of diseased cells is 1.
23. The method according to any one of items 1 to 22, wherein the copy number is the absolute copy number.
24. The method according to item 23, wherein the absolute copy number is an error corrected absolute copy number.
25. The method according to item 23 or 24, wherein the absolute copy number or the error corrected absolute copy number is normalized against a ploidy, preferably the ploidy of the genome of the diseased cell, or the ploidy of the chromosome or a portion of the chromosome on which the mutation or mutated gene is located in the diseased cell.
26. A method for determining the suitability of a neoepitope resulting from a disease-specific mutation in a gene as a disease-specific target comprising determining, in a diseased cell or population of diseased cells, whether the gene having the disease-specific mutation is an essential gene.
27. The method according to item 26, wherein the essential gene is a gene which when silenced or its expression is reduced, at least results in impaired growth or reduced fitness of a cell in which the essential gene is expressed, preferably the diseased cell.
28. The method according to item 26, wherein the essential gene is expressed in a wide variety of different tissues and is expressed with a minimal RPKM threshold greater than 0.
29. The method according to any one of items 26 to 28, wherein where the gene is an essential gene and all copies of the essential gene have the disease-specific mutation indicates the suitability of the neoepitope as a disease-specific target.
30. The method according to any one of items 26 or 29, wherein a high fraction of diseased cells contain copies of the essential gene in which all copies of the essential gene have the disease-specific mutation.
31. The method according to item 30, wherein the higher the fraction of diseased cells containing copies of the essential gene in which all copies of the essential gene have the disease-specific mutation, the higher the suitability of the neoepitope as a disease-specific target.
32. The method according to item 30 or 31, wherein the fraction of diseased cells is 1.
33. A method for determining the suitability of a combination of at least two neoepitopes resulting from disease-specific mutations in at least two genes as a combination of disease-specific targets comprising determining whether a combination of the at least two genes each having a disease-specific mutation are synthetic lethal or synthetic sick genes.
34. The method according to item 33, wherein when the combination of the at least two genes are synthetic lethal or synthetic sick indicates the suitability of the combination of neoepitopes as a combination of disease-specific targets.
35. The method according to item 33 or 34, wherein all copies of the at least two genes have the disease-specific mutations.
36. The method according to item 35, wherein a high fraction of diseased cells contain the at least two genes having the disease-specific mutations.
37. The method according to item 36, wherein the fraction of diseased cells is 1.
38. The method according to any one of items 1 to 37, wherein the disease-specific mutation is a single nucleotide variation.
39. The method according to any one of items 1 to 38, wherein the disease is cancer.
40. The method according to any one of items 1 to 39, wherein the neoepitope is identified by a method comprising sequencing the genome or a portion thereof of a diseased cell.
41. The method according to any one of items 1 to 40 for use in the manufacture of a medicament.
42. The method according to any one of items 1 to 40 for use in the manufacture of a vaccine.
43. The method according to item 42, wherein the vaccine is derived from one or more suitable neoepitopes or from a combination of suitable neoepitopes.
44. The method according to item 42 or 43, wherein the vaccine comprises a peptide or polypeptide comprising one or more suitable neoepitopes or a combination of suitable neoepitopes, or a nucleic acid encoding said peptide or polypeptide.
45. A method for providing a vaccine comprising identifying a suitable neoepitope or a combination of suitable neoepitopes according to the method of any one of items 1 to 40.
46. The method according to item 45, wherein the vaccine comprises a peptide or polypeptide comprising one or more suitable neoepitopes or a combination of suitable neoepitopes, or a nucleic acid encoding said peptide or polypeptide.
47. A vaccine produced by the method of any one of items 42 to 46.
48. The method according to any one of items 1 to 40 for use in the manufacture of recombinant immune cells expressing an antigen receptor targeted to a suitable neoepitope or to one neoepitope in a combination of suitable neoepitopes.
49. The method according to item 48, wherein the immune cells are T cells and the antigen receptor is a T cell receptor.
50. A method for providing a recombinant immune cell targeted to a suitable neoepitope or to one epitope in a combination of suitable neoepitopes, said method comprising transfecting an immune cell with a recombinant antigen receptor targeted to the suitable neoepitope or to the one epitope in a combination of suitable epitopes identified by the method according to any one of items 1 to 40.
51. The method according to item 50, wherein the immune cell is a T cell and the antigen receptor is a T cell receptor.
52. A recombinant immune cell produced by the method of any one of items 48 to 51.
53. A method for providing an immune response to a target cell population or target tissue expressing one or more neoepitopes in a mammal, said method comprising administering to the mammal:
   (a) one or more immune cells expressing one or more antigen receptors targeted to the one or more neoepitopes identified according to the method of any one of items 1 to 40;
   (b) administering a nucleic acid encoding one or more of the neoepitopes identified according to the method of any one of items 1 to 40; or
   (c) administering a peptide or polypeptide comprising one or more of the neoepitopes identified according to the method of any one of items 1 to 40.
54. The method according to item 53, wherein the immune cells are T cells and the antigen receptors are T cell receptors.
55. The method according to item 53 or 54, wherein the immune response is a T cell-mediated immune response.
56. The method according to any one of items 53 to 55, wherein the immune response is an anti-tumor immune response and the target cell population or target tissue expressing the one or more suitable neoepitopes is tumor cells or tumor tissue.
57. A method for treating a mammal having a disease, disorder or condition associated with expression of a neoepitope, the method comprising administering to the mammal:
   (a) one or more immune cells expressing one or more antigen receptors targeted to the one or more neoepitopes identified according to the method of any one of items 1 to 40;
   (b) administering a nucleic acid encoding one or more of the neoepitopes identified according to the method of any one of items 1 to 40; or
   (c) administering a peptide or polypeptide comprising one or more of the neoepitopes identified according to the method of any one of items 1 to 40.
58. The method according to item 57, wherein the immune cells are T cells and the antigen receptors are T cell receptors.
59. The method according to item 57 or 58, wherein the disease, disorder or condition is cancer.

## Claims

1. A personalized cancer vaccine for use in the treatment or prevention of a cancer in a patient, wherein the cancer of the patient is **characterized by** cancer cells comprising one or more cancer-specific neoepitopes, each neoepitope resulting from a mutation at an allele of a gene of the patient, wherein
(a) the copy number of the mutated allele encoding each neoepitope is greater than 2; or
(b) the copy number of the mutated allele encoding each neoepitope over the total number of copies of the gene is greater than 0.5, and preferably is 1,
the treatment or prevention comprising administering to the patient a vaccine comprising a peptide or polypeptide comprising the one or more cancer-specific neoepitopes or a nucleic acid, preferably a ribonucleic acid (RNA), encoding said peptide or polypeptide.

2. The personalized cancer vaccine for the use of claim 1, wherein the patient is **characterized by** a copy number of the mutated allele which is greater than 3, 4, 5, 6, 7, 8, 9, 10, 20, 30, 40, 50, 60, 70, 80, 90, or is greater than 100.

3. The personalized cancer vaccine for the use of claim 1 or 2, wherein the polypeptide is a recombinant polypeptide comprising neo-epitopes derived from different proteins or from different portions of the same protein fused together by peptide bonds or linkers.

4. The personalized cancer vaccine for the use of any one of claims 1 to 3, wherein the RNA comprises a 5'-cap or a 5'-cap analog, preferably 7-methylguanosine cap (m⁷G).

5. The personalized cancer vaccine for the use of any one of claims 1 to 4, wherein in the RNA 5-methylcytidine is substituted partially or completely for cytidine or pseudouridine is substituted partially or completely for uridine.

6. The personalized cancer vaccine for the use of any one of claims 1 to 5, wherein the RNA comprises a 5'- or 3'-untranslated region (UTR).

7. The personalized cancer vaccine for the use of any one of claims 1 to 6, wherein the RNA comprises a poly(A) sequence, preferably a poly(A) sequence having a length of 100 to 150 adenosine residues.

8. The personalized cancer vaccine for the use of any one of claims 1 to 7, wherein the RNA is associated with a carrier, preferably a lipid-containing carrier.

9. The personalized cancer vaccine for the use of claim 8, wherein the carrier comprises cationic lipids, liposomes, micelles, or nanoparticles.

10. The personalized cancer vaccine for the use of any one of claims 1 to 9, wherein the cancer is selected from the group consisting of leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the metastases thereof.

11. The personalized cancer vaccine for the use of any one of claims 1 to 10, wherein said one or more neoepitopes are T cell epitopes.

12. The personalized cancer vaccine for the use of any one of claims 1 to 11, wherein the suitability of the peptide for cancer vaccination is determined by one or more of the following:
(i) assessing whether the mutation is located in known or predicted MHC presented epitopes,
(ii) *in vitro* and/or *in silico* testing whether the mutations are located in MHC presented epitopes, and
(iii) *in vitro* testing whether the envisaged modified epitopes, when flanked by amino acid sequences also flanking said epitopes in the naturally occurring protein and when expressed in antigen presenting cells, can stimulate T cells of the patient having the desired specificity.

13. A method for determining the suitability of a neoepitope resulting from a disease-specific mutation at an allele in a gene (mutated allele) as a disease-specific target comprising determining, in a diseased cell or population of diseased cells, the copy number of the mutated allele encoding the neoepitope,
wherein a copy number of the mutated allele encoding the neoepitope over the total number of copies of the gene of greater than 0.5, and preferably of 1, indicates the suitability of the neoepitope as a disease-specific target.
